(19) Europäisches Patentamt
European Patent Office
Office européen des brevets

(11) **EP 2 775 997 B1**

(12) **EUROPÄISCHE PATENTSCHRIFT**

(45) Veröffentlichungstag und Bekanntmachung des Hinweises auf die Patenterteilung:
**07.08.2019 Patentblatt 2019/32**

(51) Int Cl.:
*A61K 8/39* (2006.01)      *A61Q 17/04* (2006.01)
*A61K 8/37* (2006.01)      *A61K 8/06* (2006.01)

(21) Anmeldenummer: **12727303.5**

(22) Anmeldetag: **05.06.2012**

(86) Internationale Anmeldenummer:
**PCT/EP2012/002370**

(87) Internationale Veröffentlichungsnummer:
**WO 2012/167901 (13.12.2012 Gazette 2012/50)**

(54) **KOSMETISCHE ODER DERMATOLOGISCHE LICHTSCHUTZZUBEREITUNG MIT VERBESSERTER WASSERFESTIGKEIT**

COSMETIC AND DERMATOLOGICAL PHOTOPROTECTIVE PREPARATION WITH IMPROVED WATER RESISTANCE

COMPOSITION COSMÉTIQUE OU DERMATOLOGIQUE DE PHOTOPROTECTION, AYANT UNE RÉSISTANCE À L'EAU AMÉLIORÉE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **07.06.2011 DE 102011077037**

(43) Veröffentlichungstag der Anmeldung:
**17.09.2014 Patentblatt 2014/38**

(73) Patentinhaber: **Beiersdorf AG**
**20253 Hamburg (DE)**

(72) Erfinder:
• **VON THADEN, Stefanie**
**20255 Hamburg (DE)**
• **KÖHLER, Manuela**
**22147 Hamburg (DE)**

(74) Vertreter: **Hartmann, Jost**
**Beiersdorf AG**
**Unnastrasse 48**
**20253 Hamburg (DE)**

(56) Entgegenhaltungen:
**EP-A1- 1 512 405       FR-A1- 2 924 020**
**GB-A- 2 450 727        US-A1- 2004 071 642**
**US-A1- 2010 209 365    US-A1- 2011 110 869**

**Beschreibung**

Die Erfindung umfasst wasserfeste kosmetische oder dermatologische Lichtschutzzubereitungen mit verbesserter Wasserfestigkeit aufgrund des Zu- bzw. Ersatzes üblicher Emulgatoren durch Polyglyceryl-10-Stearat.

Es ist bekannt, dass durch Lichtstrahlung mit einer Wellenlänge im Bereich von 280 bis 400 nm die menschliche Epidermis gebräunt werden kann und dass dies von Menschen als attraktiv empfunden wird. Andererseits ist übermäßige UV Bestrahlung für die Haut schädlich: Erytheme und Hautverbrennungen, im Volksmund auch Sonnenbrand sind die Folge. Übermäßige Sonnenbestrahlung sollte vermieden werden oder, wenn dies nicht möglich ist, durch geeignete Lichtschutzprodukte vermindert werden. Es ist ferner bekannt, dass UV-A-Strahlung mit einer Wellenlänge im Bereich von 320 bis 400 nm die Haut nachträglich nachbräunen lässt aber auch eine Veränderung der Haut hervorrufen kann, insbesondere im Falle von empfindlicher Haut oder Haut, die kontinuierlich Sonnenstrahlung ausgesetzt ist. UV-A-Strahlung bewirkt insbesondere einen Verlust der Elastizität der Haut und das Auftreten von Falten, was zu einer vorzeitigen Alterung führt. Sie begünstigt das Auslösen einer Erythembildung oder verstärkt diese Reaktion bei manchen Personen und sie kann sogar die Ursache für durch Licht ausgelöste toxische oder allergische Reaktionen sein. Es ist daher wünschenswert auch die UV-A-Strahlung auszufiltern.

Im allgemeinen ist das Lichtabsorptionsverhalten von Lichtschutzfiltersubstanzen sehr gut bekannt und dokumentiert, zumal in den meisten Industrieländern Positivlisten für den Einsatz solcher Substanzen existieren, welche recht strenge Maßstäbe an die Dokumentation anlegen. Je nachdem in welchem Bereich des UV-Lichtes absorbiert wird, unterscheidet man UV-B-Filter, UV-A-Filter und Breitbandfilter, welche über den gesamten Bereich des UV-A und UV-B eine Filterwirkung zeigen. Durch entsprechende Auswahl des UV-Filters und seiner Konzentration im Sonnenschutzmittel hat man die Möglichkeit, den Grad der Abschirmung des UV-Lichtes zu beeinflussen. Für die Dosierung der Substanzen in den fertigen Formulierungen können die Extinktionswerte allerdings allenfalls eine Orientierungshilfe bieten, denn durch Wechselwirkungen mit Inhaltsstoffen der Formulierung oder der Haut selbst können Unwägbarkeiten auftreten. Ferner ist es in der Regel schwierig vorab abzuschätzen, wie gleichmäßig und in welcher Schichtdicke die Filtersubstanz in und auf der Hornschicht der Haut verteilt ist oder wird, was für den Schutzmechanismus wiederrum essentiell ist.

Neben diesem Einfluss ist auch die Bindungsfähigkeit des UV-Filters in oder auf der Haut von großer Bedeutung für die Wasserfestigkeit der Formulierung. Es ist verständlich, dass öllösliche UV-Filter besser an die (lipophile) Oberfläche der Haut gebunden werden bzw. schwerer von dieser abwaschbar sind als wasserlösliche UV-Filter.

Die Wirksamkeit von Sonnenschutzmittel bzw. der ihnen zugrunde liegenden UV-Filter wird in der Regel in biologischen Wirksamkeitsprüfungen unter standardisierten Bedingungen bestimmt.

Der Lichtschutzfaktor LSF, oft auch SPF (sun protection factor) genannt, gibt die Verlängerung der Sonnenbestrahlung an, die durch Verwendung des Sonnenschutzmittels ermöglicht wird. Er ist der Quotient aus Erythemschwellenzeit mit Sonnenschutzmittel und Erythemschwellenzeit ohne Sonnenschutzmittel.

Der SPF (Sun Protection Factor/ Lichtschutzfaktor) wird nach den Vorschriften der International SPF Test Methode (COLIPA, Mai 2006) in vivo auf menschlicher Haut bestimmt. Für die Prüfung sind mindestens 10 Probanden erforderlich, das Konfidenz-Intervall für den Mittelwert daraus darf nicht mehr als 17% betragen.

Definition ist SPF = Quotient aus minimaler erythemaler Dosis (MED) auf geschützter und ungeschützter Haut.

Die MED ist die niedrigste Dosis von UV-Strahlung, die nach 16-24h eine schwache, aber deutliche erkennbare Hautrötung (Sonnenbrand, Erythem) hervorruft. Strahlungsquellen sind Sonnen-Simulatoren, meistens Xenon-Lampen. UV-Strahlung verschiedener Frequenz/Wellenlänge dringt unterschiedlich tief ins Gewebe ein. Daher ist auch die Art der Schädigung wellenlängenabhängig. Dem UV-A-Schutz kommt hier eine besondere Bedeutung zu, da diese UV-Strahlung (UV-A (Wellenlänge 320 - 400 nm), im Wesentlichen für die Hautalterung verantwortlich ist

UVA-Filter zählen zu den chemischen Lichtschutzfiltern. Sie nehmen die energiereiche UV-Strahlung auf und wandeln diese in Wärme- oder Lichtenergie um, welche keine Schäden mehr anrichten kann.

Zur Prüfung der UV-A-Schutzleistung kann zum Beispiel die IPD-Methode verwendet (IPD ≡ immediate pigment darkening) werden. Hierbei wird - ähnlich der Bestimmung des Lichtschutzfaktors - ein Wert ermittelt, der angibt, um wie viel länger die mit dem Lichtschutzmittel geschützte Haut mit UV-A-Strahlung bestrahlt werden kann, bis die gleiche Pigmentierung auftritt wie bei der ungeschützten Haut.

Eine andere, europaweit etablierte Prüfungsmethode ist der Australische Standard AS/NZS 2604:1997. Dabei wird die Absorption der Zubereitung im UV-A-Bereich gemessen. Um den Standard zu erfüllen, muss die Zubereitung mindestens 90 % der UV-A-Strahlung im Bereich 320 -360 nm absorbieren.

Um eine Auskunft über die UVA-Schutzleistung eines Sonnenschutzproduktes zu geben, können verschiedene Versuchsmodelle, sowohl in-vivo als auch in-vitro, angewandt werden und davon abgeleitete Aussagen auf den Verpackungen getroffen (UVA-Schutz nach Australischem Standard, Persistant Darkening Faktor u. a). Diese Angaben sind jedoch untereinander nicht vergleichbar. Aus diesem Grund hat die EU-Kommission am 22. September 2006 eine Empfehlung veröffentlicht, die für mehr Sicherheit und Transparenz bei Sonnenschutzmitteln sorgen soll. Die EU-Empfehlung sieht einen UVA-Schutz von 1/3 im Verhältnis zum UVB-Schutz vor. Bislang war lediglich der Schutz von sonnenbrandverursachenden UVB-Strahlen in der Kosmetikverordnung geregelt. Mit der neuen EU-Empfehlung soll jedes Sonnenschutzmittel gleichzeitig auch vor UVA-Strahlung schützen. Der Verbraucher soll davon ausgehen, dass ein UVA-Schutz gewährleistet ist, der mit steigendem SPF-Wert ansteigt. Aus ethischen Gründen wird dazu eine in-vitro-Methode vorgeschlagen. Bei der Beiersdorf AG in Hamburg wurde eine in-vitro Methode zur UVA-Bestimmung in Abhängigkeit an den Lichtschutzfaktor entwickelt: die UVA/UVB-Balance. Im Februar 2005 wurde die UVA/UVB-Balance als DIN 67502 veröffentlicht und ist damit die erste offizielle Methode zur Bestimmung des UVA-Schutzes im europäischen Raum. Die Methode zur Messung der UVA-Schutzleistung wird auch bei der COLIPA bearbeitet, weil die Industrie nach einer einheitlichen Methode verlangt. Die COLIPA ist der Dachverband der europäischen Kosmetikindustrie, sie gibt u.a. Empfehlungen hinsichtlich der Ausführung der Messmethode heraus. Die UVA/UVB-Balance stellt die Basis der dort diskutierten Methode dar. Sie wird dahingehend modifiziert, dass sie um eine Vorbestrahlung mit UV-Licht erweitert wird, um auch einige spezielle UV-Filtersysteme mit einbeziehen zu können. Bisher wurden die Produkte ohne Vorbestrahlung getestet. Dadurch konnte kein Aufschluss darüber gegeben werden, ob die Schutzwirkung des Produktes auch unter realen Bedingungen durch Sonneneinwirkung gegeben ist. Diese erweiterte Messmethode trägt den Namen "COLIPA Ratio".

Das bedeutet, dass UVA-Filter in Relation von 1:3 zu UVB-Filtern eingesetzt werden und damit der UVA-Schutz mit steigendem Lichtschutz angehoben wird. Die EU-Empfehlung sieht einen UVA-Schutz von 1/3 im Verhältnis zum UVB-Schutz vor. Diese Messmethode trägt den Namen "COLIPA Ratio". Die COLIPA Ratio muss der Formel nach

$$\frac{UVB}{UVA} < 3$$

sein.

Die COLIPA Ratio ist der Standard zur Bestimmung des UVA-Schutzes in Europa.

Hinsichtlich der Stabilisierung und Erreichung eines hohen UV-Schutzes gibt es vielfältige Offenbarungen im Stand der Technik.

Häufige Erscheinungsformen kosmetischer oder dermatologischer Zubereitungen sind feindisperse Mehrphasensysteme, in welchen eine oder mehrere Fett- bzw. Ölphasen neben einer bzw. mehreren Wasserphasen vorliegen. Von diesen Systemen sind am weitesten verbreitet wiederum die eigentlichen Emulsionen.

Aber auch emulgatorarme bzw. -freie Zubereitungen auf Basis sogenannter Hydrodispersionen sind bekannt. Hydrodispersionen stellen Dispersionen einer flüssigen, halbfesten oder festen inneren (diskontinuierlichen) Lipidphase in einer äußeren wäßrigen (kontinuierlichen) Phase dar. Hydrodispersionen sind - wie auch Emulsionen, die sich durch eine ähnliche Phasenanordnung auszeichnen - metastabile Systeme und daher geneigt, in einen Zustand zweier in sich zusammenhängender diskreter Phasen überzugehen. In einer klassischen O/W-Emulsion verhindert die Wahl eines geeigneten Emulgators die Phasentrennung. Im Gegensatz zur klassischen Emulsionen enthalten Hydrodispersionen aber nur sehr geringe Mengen an Emulgatoren bzw. können sogar gänzlich frei von Emulgatoren sein.

Als kosmetische oder medizinische Zubereitungen sind oftmals Emulsionen, insbesondere W/O-, O/W-, O/W/O oder W/O/W-Emulsionen, im Einsatz. Unter Emulsionen versteht man im Allgemeinen heterogene Systeme, die aus zwei nicht oder nur begrenzt miteinander mischbaren Flüssigkeiten bestehen, die üblicherweise als Phasen bezeichnet werden. In einer Emulsion ist eine der beiden Flüssigkeiten (W/O) in Form feinster Tröpfchen in der anderen Flüssigkeit dispergiert. Die Flüssigkeiten (rein oder als Lösungen) liegen in einer Emulsion in einer mehr oder weniger feinen Verteilung vor, die im Allgemeinen nur begrenzt stabil ist.

Sind die beiden Flüssigkeiten Wasser und Öl und liegen Öltröpfchen fein verteilt in Wasser vor, so handelt es sich um eine Öl-in-Wasser-Emulsion (O/W-Emulsion, z. B. Milch). Der Grundcharakter, zum Beispiel elektrische Leitfähigkeit, Sensorik, Anfärbbarkeit der kontinuierlichen Phase, einer O/W-Emulsion ist durch das Wasser geprägt. Bei einer Wasser-in-Öl-Emulsion (W/O-Emulsion, z. B. Butter) handelt es sich um das umgekehrte Prinzip, wobei der Grundcharakter hier durch das Öl bestimmt wird.

Der Stand der Technik kennt mehrere wesentliche Faktoren, die einen positiven Einfluss auf die Stabilität und Rheologie von Emulsionen haben.

Emulsionen benötigen zu ihrer Bildung und zur Stabilisierung im Allgemeinen einen oder mehrere Emulgatoren, Verdicker und/oder Konsistenzgeber, um über einen kosmetisch akzeptablen Zeitraum stabil zu sein, im Allgemeinen 1 Jahr nach dem Öffnen einer kosmetischen Zubereitung.

Dazu werden oft große Mengen an Emulgatoren benötigt. Dies wiederum kann bei Konsumenten zu Missempfindungen bis zur Unverträglichkeit und im Extremfall sogar zu Phänomene wie die "Mallorca Akne" o.ä. führen.

Wünschenswert ist es daher Emulsionszubereitungen zur Verfügung zu stellen, die möglichst geringe Mengen an Emulgatoren umfassen und dennoch ausreichend stabil formuliert sind.

Die Wasserfestigkeit von Lichtschutzzubereitungen ist eine weitere Herausforderung an den Kosmetikfachmann. Die Wasserfestigkeit einen Lichtschutzproduktes ist essentiell. Insbesondere am Strand möchte man nicht nach jedem Bad im Meer oder nach jeder Dusche sich erneut eincremen müssen.

O/W-Emulsionen sind leicht verstreichbar und auch bei fettiger Haut und Akne geeignet. Ein Nachteil ist oft, insbesondere bei leicht einziehenden Formulierungen, die geringe Wasserfestigkeit.

W/O-Emulsionen sind für trockene Haut gut geeignet, haben eine hohe Wasserfestigkeit und sind gut mit Pigmenten kombinierbar. Sie sind aber klebrig, ziehen weniger gut ein und sind deshalb bei einigen Anwendern nicht beliebt.

Hydrodispersionsgele sind leicht verstreichbar und führen zu einem angenehmen Hautgefühl. Sie können ohne Emulgatoren und Konservierungsmittel hergestellt werden und sind daher gut geeignet für Personen mit Sonnenallergie. Sie sind für behaarte Hautstellen und bei fettiger Haut und Akne geeignet, haben aber eine geringe Wasserfestigkeit.

Sonnenschutzmittel in Sprays sind zwar leicht verteilbar, haben aber meist eine geringe Wasserfestigkeit.

Wasserfeste Lichtschutzformulierungen werden üblicherweise durch Verwendung von einem oder mehreren der in Lichtschutzformulierungen üblichen Filmbildnern (u.a. Unimer- oder Antarontypen sowie Synchrowachse) erzielt. Diese Systeme zeichnen sich aber durch eine für den Verbraucher unattraktive Sensorik aus, d.h. sie sind häufig insbesondere stumpf, klebrig und ölig auf der Haut und ziehen schlecht ein.

Diesen Umstand zu verbessern ist eine weitere Aufgabe der vorliegenden Erfindung.

Es gibt viele Emulgatoren mit denen die Wasserfestigkeit nicht negativ beeinflusst wird. Zum Beispiel die Fettsäureester, wie Glyceryl Stearate Citrate (Imwitor® von Sasol), beeinflussen die Wasserfestigkeit nicht negativ. Wie zuvor ausgeführt, beeinflussen jedoch alle Emulgatoren die Sensorik der Lichtschutzzubereitungen. Emulgatoren, die für die Wasserfestigkeit positiv wirken, sind tendenziell lipophil und die resultierende Sensorik ist eher reichhaltig, schwer und/oder klebrig. Greift der Fachmann jedoch zu hydrophilen Emulgatoren wird die Sensorik leicht und nicht klebrig. Diese Sensorik wird vom Konsumenten bevorzugt.

Problem dabei ist jedoch, dass hydrophile Emulgatoren die Wasserfestigkeit negativ beeinflussen, weil der hydrophile Emulgator aufgrund seiner Hydrophilie leicht abgewaschen wird und dabei in seiner Eigenschaft als Emulgator die UV Filter mitnimmt. Die Eigenschaften "leichte Formulierung durch hydrophilen Emulgator" und "schlechte Wasserfestigkeit durch hydrophilen Emulgator" scheinen nicht mit dem Konsumentenwunsch nach einer leichten und wasserfesten Formel vereinbar. Dies scheint ein quasi unlösbaren Problem, man muss sich zwischen einer leichten Sensorik oder der Wasserfestigkeit entscheiden.

Weiterhin müssen kosmetische oder dermatologische Zubereitungen einigen ästhetischen Gesichtspunkten genügen um eine ausreichende Verbraucherakzeptanz zu erlangen.

Dem Fachmann sind Emulgatoren aus dem Lebensmittelbereich bekannt wie Glyceryl Stearate Citrate (Imwitor®) oder Polyglyceryl-10-Stearat.

Auf der Suche nach geeigneten Emulgatoren, die den gewünschten Anforderungen der Kosmetik und Dermatologie entsprechen, scheinen diese Emulgatoren sich aber auszuschließen.

Glyceryl Stearate Citrate muss in kosmetischen Zubereitungen in einer solch hohen Menge oder in Kombination mit Co-Emulgatoren eingesetzt werden um stabile Emulsionen zu erhalten, so dass der Wunsch nach verringertem Emulgatorgehalt mit Glyceryl Stearate Citrate nicht zu erfüllen ist.

Polyglyceryl-10-Stearat (PG-10-Stearat, CAS-Nr.: 79777-30-3) ist ein hydrophiler Emulgator und weist einen HLB von 12 auf.

Im Handel sind Polyglyceryl-10-Stearate als Mischungen beispielsweise erhältlich als Polyaldo 10-1-S (Lonza), Nikkol Decaglyn 1-S (Nikko Chemicals Co., Ltd.) oder Salacos PGMSV (The Nisshin OilliO Group, Ltd.).

Polyglyceryl-10-Stearat ist aus der Eiscremeherstellung bekannt und weist eine braune Farbe auf. Auch aufgrund dieses ästhetisch problematischen Umstandes schien sich die Anwendung in kosmetischen Emulsionen von selbst zu verbieten.

Käuflich erwerbar ist Heliogel®, welches neben Sodium Acrylates Copolymer, Hydrogenated Polyisobutene, Phospholipids, Helianthus Annuus (Sunflower) Seed Oil auch Polyglyceryl-10-Stearate umfasst.

In kosmetischen Zubereitungen ist PG-10-Stearat lediglich als Bestandteil eines Aftershavegels genannt. In dieser Zubereitung sind 6 Gew.% PG-10-Stearat neben weiteren Emulgatoren wie Lecithin und PEG 150 distearat beschrieben (WO 2003082182).

JP 2005162664 offenbart PG-10-stearat in Haarfärbezubereitung umfassend Peroxide. Eine kosmetische topische Applikation schließt sich daher aus.

US 5925615 offenbart PG-10-stearat in Shampooformulierungen umfassend Polyethylenglykole und Parabene. DE 19547679 A1 beschreibt eine Haarpflegeemulsionen mit PG-10-stearat.

DE 19724587 A1 beschreibt eine Haarspülung mit PG-10-stearat sowie Polyethylenglykole und Parabene.

WO 0027197 A1 offenbart Zubereitungen umfassend neben PG-10-stearat ein oder mehrere UV Filter, Lecithine, Polyethylenglykole und Parabene.

DE 102008028821 A1 und DE 102008028822 A1 offenbaren Stiftzubereitungen mit PG-10-stearat.

DE 10213955 A1 offenbart PG-10-stearat in einer After Shave Lotion, wobei der Gehalt an PG-10-stearat 6% und mehr beträgt.

US 20060018853 beschreibt peel-off Zubereitung mit PG-10-stearat mit Decaglyceryltriisostearat und Lecithin.

Überraschenderweise zeigte sich jedoch, dass sich Polyglyceryl-10-Stearat als Emulgator in kosmetischen oder dermatologischen Zubereitungen als Emulgator einsetzen lässt, ohne die Nachteile der färbenden Wirkung und der Instabilität zu zeigen.

Durch Zusatz von PG-10-Stearat anstelle anderer Emulgatoren wird überraschenderweise dabei auch die Wasserfestigkeit verbessert. Dies ist umso erstaunlicher, da es sich bei Polyglyceryl-10-Stearat (PG-10-Stearat) um einen hydrophilen Emulgator handelt.

Besonders überraschend ist, dass PG-10-Stearat sowohl als alleiniger Emulgator als auch in Emulgatormischungen einsetzbar ist und die Lichtschutzzubereitungen eine verbesserte Wasserfestigkeit zeigen.

Die Erfindung ist daher eine wasserfeste kosmetische oder dermatologische Lichtschutzzubereitung umfassend ein oder mehrere UV-Lichtfiltersubstanzen und Polyglyceryl-10-Stearat.

Der Anteil an Polyglyceryl-10-Stearat in der Lichtschutzzubereitung ist mit 0,1 bis weniger als 6 Gew. %, bezogen auf die Gesamtmasse der Zubereitung, zu wählen. Insbesondere bevorzugt ist der Anteil an PG-10-Stearat weniger als 3 Gew. %, insbesondere im Bereich von 0,1 bis 2,8 Gew.%, bezogen auf die Gesamtmasse der Zubereitung, zu wählen.

Der Vorteil hierbei ist, dass der Wunsch nach geringen Emulgatorgehalten genüge getan wird.

Ausgenommen von den erfindungsgemäßen Zubereitungen sind O/W-Emulsionszubereitungen 4 und 5 bestehend aus

| | 4 | 5 |
|---|---|---|
| Panthenol | 0,7 | 0 |
| Butylenglykol | 5 | 10 |
| Propylenglykol | 5 | --- |
| Benzethoniumchlorid | 0,5 | 0,9 |
| Lauroylethylarginat | 1,0 | 0,5 |
| medizinisches Weissöl | 0 | 0 |
| Isopropylpalmitat | 0 | 0 |
| Caprylic/Capric Triglycerid | 0 | 1,00 |
| Cetearylalkohol | 2,5 | 4,00 |
| Cetylalkohol | 0 | 0 |
| lineares Silikonöl | 0 | 0,50 |
| cyclisches Silikonöl | 0 | 0 |
| C12-15 Alkylbenzoat | 0 | 2,00 |
| Sheabutter | 0 | 0 |
| Dicaprylylether | 10 | 0 |
| Dicaprylylcarbonat | 0 | 0 |
| Glyceryl stearat | 2,4 | 2,4 |
| Tapiocastärke | 0 | 0 |
| Glycerin | 8 | 8 |
| Butylenglykol | 1 | 0 |
| Zitronensäure | 0 | 0 |
| Natronlauge 45% | 0,25 | 1 |
| Polyglyceryl-10 Myristat | 0 | 0 |
| Polyglyceryl-10 Stearat | 1 | 1 |
| Polyacrylsäure, Na-Salz (Carbopol 981) | 0 | 0 |
| Acrylic Acid/VP Crosspolymer | 0,5 | 0,75 |

(fortgesetzt)

|  | 4 | 5 |
|---|---|---|
| Trinatrium EDTA |  | 1 |
| Ethylhexylmethoxycinnamat | 2 | 0 |
| Butylmethoxydibenzoylmethan | 0 | 2 |
| Phenylbenzimidazolesulfonsäure, Natrium-Salz | 0 | 2 |
| Ethylhexylsalicylat | 0 | 2 |
| Titandioxid, silikongecoated | 0,3600 | 0 |
| Octocrylen | 0 | 2 |
| Parfum | 0 | 0,20 |
| Wasser | ad 100 | ad 100 |

sowie O/W-Emulsionszubereitungen 49 und 50 bestehend aus

|  | 49 | 50 |
|---|---|---|
| Panthenol | 0,7 | 0 |
| Butylenglykol | 5 | 10 |
| Propylenglykol | 5 | --- |
| Methylisothiazolinone | 0,06 | --- |
| Benzethoniumchlorid | 0,15 | --- |
| Piroctonolamin | 0,15 | --- |
| Lauroylethylarginat | 0,15 | 1,75 |
| medizinisches Weißöl | 0 | 0 |
| Isopropylpalmitat | 0 | 0 |
| Caprylic/Capric Triglycerid | 0 | 1,00 |
| Cetearylalkohol | 2,5 | 4,00 |
| Cetylalkohol | 0 | 0 |
| lineares Silikonöl | 0 | 0,50 |
| zyklisches Silikonöl | 0 | 0 |
| C12-15 Alkylbenzoat | 0 | 2,00 |
| Sheabutter | 0 | 0 |
| Dicaprylylether | 10 | 0 |
| Dicaprylylcarbonat | 0 | 0 |
| Glyceryl Stearate | 2,4 | 2,4 |
| Tapiocastärke | 0 | 0 |
| Glycerin | 8 | 8 |
| Zitronensäure | 0 | 0 |
| Natronlauge 45%ig | 0,25 | 1 |
| Polyglyceryl-10 Myristat | 0 | 0 |
| Polyglyceryl-10 Stearat | 1 | 1 |

(fortgesetzt)

| | 49 | 50 |
|---|---|---|
| Polyacrylsäure, Na-Salz | 0 | 0 |
| Acrylic Acid/VP Crosspolymer | 0,5 | 0,75 |
| Trinatrium EDTA | | 1 |
| Ethylhexylmethoxycinnamat | 2 | 0 |
| Butylmethoxydibenzoylmethan | 0 | 2 |
| Phenylbenzimidazolesulfonsäure | 0 | 2 |
| Ethylhexylsalicylat | 0 | 2 |
| Titandioxid+Trimethoxycaprylylsilan | 0,3600 | 0 |
| Octocrylen | 0 | 2 |
| Parfum | 0 | 0,20 |
| Wasser | ad 100 | ad 100 |

Die Zahlenwerte stellen Gewichtsanteile bezogen auf die Gesamtmasse der Zubereitungen dar.
Die erfindungsgemäßen Zubereitungen basieren vorteilhaft auf einer Emulsion, bevorzugt einer O/W-Emulsion, oder einer Hydrodispersion. Die so hergestellten Zubereitungen, bevorzugt Emulsionen, weisen eine niedrigen Emulgatorgehalt von weniger als 6% auf und erfüllen somit den Wunsch nach gut verträglichen, milden Produkten, ohne das die Stabilität beeinträchtigt wird. Der Mindestanteil an PG-10-Stearat beträgt 0,1 Gew.%. Überraschenderweise sind die mit weniger als 6 Gew.% Polyglyceryl-10-Stearat hergestellten Zubereitungen stabil für einen Zeitraum von mindestens 3 Jahre bei Raumtemperatur und mindestens 6 Monate bei 40°C Lagerung.
Des Weiteren zeigte sich überraschend, dass Polyglyceryl-10-Stearat, auch als einziger Emulgator eingesetzt, zu stabilen Emulsionen führt. Ein Verzicht auf weitere zusätzliche Emulgatoren ist damit möglich und kommt so dem Wunsch nach verringerten Emulgatorgehalten nach.
Filmbildner sind als Stoffe zur Erzielung der Wasserfestigkeit von Zubereitungen bekannt. Diese sind durch folgende Eigenschaft charakterisiert: Löst man einen Filmbildner in Wasser oder anderen geeigneten Lösemitteln und trägt die Lösung dann auf die Haut auf, so bildet sich nach dem Verdunsten des Lösemittels ein Film aus, der im Wesentlichen dazu dient die Lichtschutzfiltersubstanzen auf der Haut zu fixieren und vor allem die Wasserfestigkeit der Zubereitung auf der Haut zu gewährleisten bzw. zu steigern.
Bekannte Filmbildner sind beispielsweise Polymere auf Basis von PVP (Antaron V216, V220 der GAF ChemicalsCooperation), Natriumpolystyrensulfonat (Flexan 130 der National Starch) oder Polyisobuten (Rewopal PIB 1000). Weitere für Lichtschutzprodukte typische Filmbildner, die die Wasserfestigkeit verbessern, sind zum Beispiel Unimer- oder Antarontypen und Synchrowachse.
Die erfindungsgemäßen Zubereitungen weisen gegenüber Zubereitungen ohne PG-10-Stearat aber mit gleichem UV-Filteranteil und Filmbildnern eine verbesserte Wasserfestigkeit auf.
Die Wasserfestigkeit von Sonnenschutzprodukten kann nach den Vorschriften der COLIPA WR (Water Resistance) Methode von Dezember 2005 in vivo auf menschlicher Haut oder über die Kontaktwinkelmethode bestimmt werden.
Die SPF-Bestimmung erfolgt einmal auf trockener Haut (static) und ein zweites Mal nach mindestens 2 x 20 Minuten Wässerung und 15 min Trockenzeit (2x20 min = water resistant, 4x20 min = extra water resistant). Die Wasserfestigkeit wird bestätigt, wenn der Wert nach Wässerung nach Abzug des Konfidenzintervalls größer 50% des SPF static ist.
Neben diesem in vivo Test gibt es auch einen in vitro Test über die Kontaktwinkelmethode (Contact angle measurement-a reliable supportive method for screening water-restistance of ultraviolet-protecting products in vivo" Intern. Journal of Cosmetic Science, 2007, 29, 283-291).
Entsprechend dieser Methode wurden die Zubereitungen der Tabelle 1 untersucht.

Tabelle 1: Wasserfestigkeit kosmetischer Lichtschutzzubereitungen

| INCI | 1 | 2 |
|---|---|---|
| Xanthan Gum | 0.4000 | 0.4000 |
| Tocopheryl Acetate | 0.0600 | 0.0600 |

(fortgesetzt)

| INCI | 1 | 2 |
|---|---|---|
| Cetyl Alcohol | 2.2000 | - |
| Octyldodecanol | 5.5000 | 5.5000 |
| Myristyl Myristate | 1.5000 | 1.5000 |
| C12-15 Alkyl Benzoate | 6.5000 | 6.5000 |
| Butylene Glycol Dicaprylate/Dicaprate | 2.5000 | 2.5000 |
| Cetearyl Alcohol | - | 2.5000 |
| Cetearyl Alcohol + PEG-40 Castor Oil + Sodium Cetearyl Sulfate | 2.0000 | - |
| Glyceryl Stearate SE | 0.6500 | - |
| Glyceryl Stearate | - | 0.8500 |
| Tapioca Starch + Aqua | 1.0000 | 1.0000 |
| Parfum | 0.4000 | 0.4000 |
| Glycerin | 0.9000 | 0.9000 |
| Sodium Hydroxide aq | 0.6000 | 0.6000 |
| Polyglyceryl-10-Stearat | - | 2.5500 |
| Phenoxyethanol | 0.2000 | 0.2000 |
| Methylparaben | 0.3000 | 0.3000 |
| Acrylates/C10-30 Alkyl Acrylate Crosspolymer | 0.0500 | 0.0500 |
| Aqua | Ad 100 | Ad 100 |
| Alcohol Denat. | 8.0000 | 8.0000 |
| Aqua + Trisodium EDTA | 1.0000 | 1.0000 |
| Ethylhexyl Methoxycinnamate + BHT | 0.5000 | 0.5000 |
| Octocrylene | 4.5000 | 4.5000 |
| Bis-Ethylhexyloxyphenol Methoxyphenyl Triazine | 3.5000 | 3.5000 |
| Butyl Methoxydibenzoylmethane | 4.5000 | 4.5000 |
| Titanium Dioxide + Trimethoxycaprylylsilane | 4.0000 | 4.0000 |
| Phenylbenzimidazole Sulfonic Acid | 2.0000 | 2.0000 |
| Diethylhexyl Butamido Triazone | 1.0000 | 1.0000 |
| Kontaktwinkel | 24,8 | 61,0 |

Durch den Austausch der Emulgatoren Cetylalkohol und Ceterayl Alkohol (PEG 40 Castor Oil, Sodium Cetearylsulfat) durch PG-10-Sterate wird der Kontaktwinkel von 24.8° auf 61° erhöht.

In der Literatur wird der Kontaktwinkel als Indikator für die Wasserfestigkeit einer kosmetischen Formulierung genannt. Man geht davon aus, dass ein Winkel > 35° ein Indikator für eine wasserfeste Formel. Erfindungsgemäße wasserfeste Zubereitungen weisen demnach einen Kontaktwinkel von größer 35° auf (Kontaktwinkelmethode). Somit wird durch den Austausch des Emulgatorsystems erfindungsgemäß erst eine wasserfeste Formel erhalten. Ein solcher Effekt kann normalerweise nur durch Filmbildner erreicht werden, allerdings mit all den bereits genannten ungewollten Nachteilen. Dies kann nun erfindungsgemäß vermieden werden.

Die Untersuchungen zeigen in den Formeln 1 und 2 Carbomer und Xanthan Gum lediglich zur Viskositätseinstellung, also keine klassischen Filmbildner. Die wasserfestigkeitsverbesserte PG-10-Stearat Formel 2 enthält ebenfalls keine Filmbildner. Die Wasserfestigkeit wurde also lediglich durch den Austausch des Emulgatorsystems erreicht.

Polyglyceryl-10-Stearat lässt sich daher als Emulgator in Lichtschutzzubereitungen umfassend ein oder mehrere UV-Filtersubstanzen und gegebenenfalls ein oder mehrere Filmbildner zur Verbesserung der Wasserfestigkeit gegenüber den gleichen Zubereitungen ohne Polyglyceryl-10-Stearat verwenden.

Vorteilhaft umfassen die erfindungsgemäßen Zubereitungen ein oder mehrere Filmbildner zur weiteren Einstellung der Wasserfestigkeit

Weiterhin zeigte sich überraschenderweise, dass PG-10-Stearat bei der Emulsionsbildung sowohl in die Wasser- als auch in die Fettphase gegeben werden kann. In beiden Fällen wird das gleiche Produkt erhalten. Die Abweichungen in der Konsistenz sind nicht signifikant und für einen Konsumenten nicht relevant.

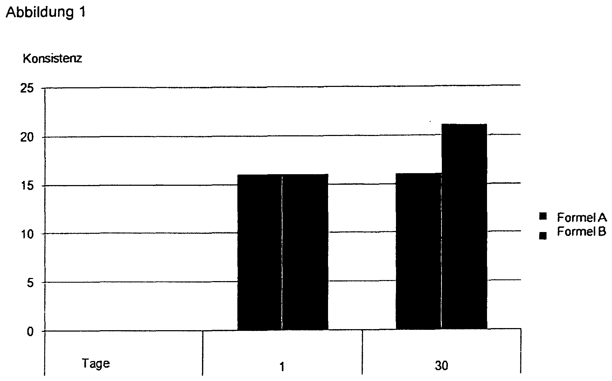

Abbildung 1 zeigt diese Konsistenzuntersuchungen. Die Konsistenz wurde mit einem Konsistenzbestimmungsgerät nach Fligge (gemäß DE 29 09 087) ermittelt.

Auf der X-Achse ist zum Einen der Wert der Konsistenz der zu vergleichenden Formeln A und B nach einem Tag (links) und nach 30 Tagen (rechts) aufgetragen. Die Formel A unterscheidet sich von der Formel B darin, dass der Emulgator PG-10-Stearate im Fall A in die Wasserphase und im Fall B in die Fettphase gegeben wurde. Alle weiteren Schritte waren für beide Formulierungen identisch.

Auf der y- Achse ist der Konsistenzwert aufgetragen. Man erkennt, dass beide Formeln nach 1 Tag sogar numerisch die gleiche Konsistenz haben, also identisch sind bezüglich der Konsistenz. Nach 30 Tagen gibt es zwar einen numerischen Unterschied, der jedoch vom Konsumenten nicht nachvollziehbar ist.

Die Untersuchungen belegen die außergewöhnlichen Eigenschaften von PG-10-Stearat. Da es nicht relevant ist, in welche Phase man den Emulgator gibt, hat man in der Phasenaufteilung in der Produktion größere Flexibilität. Ein Verfahren zur Herstellung kosmetischer oder dermatologischer Emulsionszubereitungen mit Polyglyceryl-10-Stearat als Emulgator, bevorzugt einzigen Emulgator, ermöglicht die Zugabe sowohl in die Wasser- und/oder in die Ölphase der Emulsion.

PG-10-sterat haltige Zubereitungen lassen sich somit einfacher herstellen und geben dem Fachmann eine hervorragende Flexibilität bezüglich Viskosität, Sensorik, Applikationsform und Toleranz gegenüber verschiedenster Inhaltsstoffe und der Produktion.

Den Einsatz von PG-10-Stearat in Kosmetika auf möglichst geringe Anteile zu begrenzen mag für einen Kosmetikfachmann aufgrund der braunen Farbe des PG-10-Stearats nahe gelegen haben, jedoch müsste er aufgrund der geringen Anteile dann auch von einer verminderten emulgierenden Wirkung ausgehen. Dies hat sich bei einer Verwendung von weniger als 6 Gew.% PG-10-Stearat, insbesondere im Bereich von 0,1 bis 2,8 Gew.%, bezogen auf die Gesamtmenge der Zubereitung, überraschenderweise aber nicht bewahrheitet.

In mehreren Langzeitstabilitätsuntersuchungen zeigten die PG-10-Stearat haltigen Zubereitungen keine Instabilitäten, Phasentrennung, Koaleszenz, Ostwald-Reifung oder Veränderung der Tröpfchengröße mit der Zeit oder Aufrahmung. Die Formulierungen, wie sie in den Beispielen offenbart sind, waren mindestens 6 Monate bei Raumtemperatur sowie bei 40°C optisch stabil. Das heißt es wurde keine Phasentrennung oder Phasenabscheidung beobachtet.

Neben der verbesserten Wasserfestigkeit weisen die erfindungsgemäßen Zubereitungen zum Unterschied zum Stand der Technik eine gute Produktsensorik auf und die UV-Lichtschutzleistung ist nicht eingeschränkt.

Den erfindungsgemäßen Zubereitungen können zusätzliche Emulgatoren zugesetzt werden. Dies ist aber erfindungsgemäß nicht in allen Fällen erforderlich oder zwingend.

Wenn zusätzliche Emulgatoren hinzugefügt werden, sind diese bevorzugt zu wählen aus der Gruppe der Emulgatoren, die bei 25°C fest, pastös oder flüssig und nicht ethoxiliert sind. Besonders bevorzugte zusätzliche Emulgatoren sind zu wählen aus der Gruppe 1,2-Propandiolfettsäureester, Acetoglyceride, acetylierte Mono-/Diglyceride, Alkali- bzw. Ammonium-Seifen, Ammoniumphosphatide, Sorbitanmonoisostearat, C10-C22 Fettsäuren, Cetearyl Sulfat, Cetearylglucoside, Cetylphosphat, Cetylstearylalkohol in Kombination mit Natrium Cetylstearylsulfat, Citroglyceridester, Citronensäuremonoglyceride, Diacetylweinsäuremonoglyceride, Diisostearoyl Polyglyceryl-3 Diisostearat (Isolan PDI), Emulgator YN (Handelsbezeichnung), Essigsäuremonoglyceride, gemischte Propylenglykol-Glycerinester, Glyceryllaurat, Glycerylmyristat, Glyceryloleat in Kombination mit Propylenglycol, Glycerylstearat, Glycerylstearat SE, Glycerylstearatcitrat, Glycol Distearat, Isostearyldiglycerylsuccinat, Isostearylglycerylether, Kaliumcetylphosphat, Lactoglyceride, lactylierte Mono-/Diglyceride, Methylglucosesesquistearat, Milchsäuremonoglyceride, Mono- und Diglyceride von Speisefettsäuren verestert mit Essigsäure und Weinsäure, Monoglyceridacetat, Monoglyceridcitrat, Monoglyceriddiacetyltartrat, Monoglyceridlactat, Monoglyceridtartrat, Na-,K- und Ca-Salze der Stearoyl-2-lactylmilchsäure, Na-,K- und Ca-stearoyl-2-lactoyl-lactat, Na-,K- und Ca-stearoyl-2-lactyllactat, Na-,K- und Ca-stearoylmilchsäure, Na-Salz der Laurylschwefelsäure, Natriumcetylphosphat, Natriumcetylstearylsulfat, Natriumdodecylsulfat und/oder Dodecylhydrogensulfat, Polyglycerin-3-Diisostearat (LameformTGI), Polyglycerinester der interesterifizierten Ricinolsäure, Polyglycerinfettsäureester, Polyglycerin-Polycrinoleat, Polyglyceryl Dimerate Isostearat, Polyglyceryl Polyricinoleat (Admul WOL 1403), Polyglyceryl-1 Dipolyhydroxystearat (Dehymuls PGPH), Polyglyceryl-2-laurat, Polyglyceryl-2-sesquiisostearat, Polyglyceryl-3 Beeswax (Cera Bellina), Polyglyceryl-3 Cetyl Ether (Chimexane NL), Polyglyceryl-3 Distearat (Cremophor GS 32), Polyglyceryl-3 Methylglucose Distearat (Tego Care 450), Polyglyceryl-3 Oleat, Polyglyceryl-3-methylglycosedistearat, Polyglyceryl-4 Caprat (Polyglycerol Caprate T2010190), Polyglyceryl-4 Diisostearat/Polyhydroxystearat/Sebacate (Isolan GPS), Polyglyceryl-4 Isostearat (Isolan GI 34), Polyoxyethylen-stearinsäureester, Polyoxylstearat-Mono-/Diglycerid, Propylenglycolstearat SE, Propylenglykolfettsäureester, Propylenglykolmono-/di-fettsäureester, Saccharoglyceride, Saccharoseester von Speisefettsäuren, Sorbate bsp. Sorbitanmonolaurat (Sorbat 20), Sorbitandicitrat, Sorbitandierucat, Sorbitandihydroxystearat, Sorbitandiisostearat, Sorbitandimaleat, Sorbitandioleat, Sorbitandiricinoleat, Sorbitanditartrat, Sorbitanmonocitrat, Sorbitanmonoerucat, Sorbitanmonohydroxystearat, Sorbitanmonomaleat, Sorbitanmonoricinoleat, Sorbitanmonostearat (Sorbat 60), Sorbitanmonotartrat, Sorbitansesquicitrat, Sorbitansesquierucat, Sorbitansesquihydroxystearat, Sorbitansesquiisostearat, Sorbitansesquimaleat, Sorbitansesquioleat, Sorbitansesquiricinoleat, Sorbitansesquitartrat, Sorbitantricitrat, Sorbitantrierucat, Sorbitantrihydroxystearat, Sorbitantriisostearat, Sorbitanmonooleat, Sorbitantrimaleat, Sorbitantrioleat, Sorbitantriricinoleat, Sorbitantristearat (Sorbat 65), Sorbitantritartrat, Stearinsäure sowie ihrer Salze, Sucroester, Triethylcitrat, Weinsäureglyceride, Weinsäuremonoglyceride und/oder Zuckerfettsäureester, besonders bevorzugt ist Glycerylstearat.

Bevorzugt umfassen die erfindungsgemäßen Zubereitungen keinen weiteren zusätzlichen Emulgator ausser PG-10-Stearat. Der Anteil an zusätzlichen Emulgatoren sollte somit bevorzugt unter 0,01 Gew.% liegen, bezogen auf die Gesamtmasse der Zubereitung, um als erfindungsgemäß - ohne zusätzlichen Emulgator - zu gelten.

Lecithine ist der klassische Name für eine Gruppe chemischer Verbindungen, die so genannten Phosphatidylcholine. Dabei handelt es sich um Phospholipide, die sich aus Fettsäuren, Glycerin, Phosphorsäure und Cholin zusammensetzen. Lecithine sind Bestandteile der Zellmembran tierischer und pflanzlicher Lebewesen. Sie sind Begleitstoffe in Fetten und Ölen. Sie ermöglichen das Emulgieren von Fetten und Wasser und sind somit wichtige natürliche Tenside (Emulgatoren) für Nahrungs- und Futtermittel. Lecithine sind in der EU als Lebensmittelzusatzstoff (E 322) für Lebensmittel allgemein zugelassen mit Höchstmengenbeschränkung ausschließlich bei Säuglingsnahrung. In der Medizin und in der Kosmetik werden sie auch als Wirkstoff, in der Diätetik als Nahrungsergänzungsmittel genutzt. Den Lecithinen werden neben ihren strukturbildenden Eigenschaften zahlreiche funktionelle Aufgaben zugeschrieben. Sie sind sowohl am anabolen Lipidstoffwechsel als auch am katabolen Fettstoffwechsel aktiv beteiligt.

Lecithine sind daher erfindungsgemäß auszuschließen.

Es ist erfindungsgemäß von Vorteil, wenn die Zubereitung in der Fettphase und/oder in der wässrigen Phase UV-Filter enthalten. Dabei ist es erfindungsgemäß bevorzugt, wenn als UV-Filter eine oder mehrere Verbindungen gewählt aus der Gruppe 1-(4'-tert.Butylphenyl)-3-(4'-methoxyphenyl)propan-1,3-dion, 1,4-di(2-oxo-10-Sulfo-3-bornylidenmethyl)-Benzol und dessen Salze, 1-Phenyl-3-(4'-isopropylphenyl)propan-1,3-dion, 2-(2H-benzotriazol-2-yl)-4-methyl-6-[2-methyl-3-[1,3,3,3-tetramethyl-1-[(trimethylsilyl)oxy]disiloxanyl]propyl]-phenol, 2-(4'-Diethylamino-2'-hydoxybenzoyl)-benzoesäurehexylester, 2,2'-Methylen-bis-(6-(2H-benzotriazol-2-yl)-4-(1,1,3,3-tetramethylbutyl)-phenol), 2,2'-Dihydroxy-4-methoxybenzophenon, 2,4,6-Tris-(biphenyl)-1,3,5-triazin, 2,4-Bis-(4'-Di-neopentylaminobenzalmalonat)-6-(4"-butylaminobenzoat)-s-triazin, 2,4-bis-[5-1(dimethylpropyl)benzoxazol-2-yl-(4-phenyl)-imino]-6-(2-ethylhexyl)-imino-1,3,5-triazin mit der (CAS Nr. 288254-16-0), 2,4-Bis-{[4-(1',1',1',3',5',5',5'-Heptamethylsiloxy-2-methyl-propyloxy)-2-hydroxy]-phenyl}-6-(4-methoxyphenyl)-1,3,5-triazin, 2,4-Bis-{[4-(2-ethyl-hexyloxy)-2-hydroxy]-phenyl}-6-(1-methyl-pyrrol-2-yl)-1,3,5-triazin, 2,4-Bis-{[4-(2-ethyl-hexyloxy)-2-hydroxy]-phenyl}-6-(4-methoxyphenyl)-1,3,5-triazin (INCI: Bis-Ethylhexyloxyphenol Methoxyphenyl Triazin), 2,4-Bis-{[4-(2-ethyl-hexyloxy)-2-hydroxy]-phenyl}-6-[4-(2-methoxyethylcarboxyl)-phenylamino]-1,3,5-triazin, 2,4-Bis-{[4-(2-methylpropenyloxy)-2-hydroxy]-phenyl}-6-(4-methoxyphenyl)-1,3,5-triazin, 2,4-Bis-{[4-(3-(2-propyloxy)-2-hydroxy-propyloxy)-2-hydroxy]-phenyl}-6-(4-methoxyphenyl)-1,3,5-triazin, 2,4-Bis-{[4-(3-(2-propyloxy)-2-hydroxy-propyloxy)-2-hydroxy]-phenyl}-6-[4-(ethylcarboxyl)-phenylamino]-1,3,5-triazin, 2,4-Bis-{[4-(3-sulfonato)-2-hydroxy-propyloxy)-2-hydroxy]-phenyl}-6-(4-methoxyphenyl)-1,3,5-triazin Natriumsalz, 2,4-Bis-{[4-tris(trimethylsiloxysilylpropyloxy)-2-hydroxy]-phenyl}-6-(4-methoxyphenyl)-1,3,5-triazin, 2-Ethylhexyl Methoxycinnamate, 2-Ethylhexyl-2-cyano-3,3-diphenylacrylat (Octocrylen), 2-Ethylhexyl-2-hydroxybenzoat, 2-Hydroxy-4-methoxy-4'-methylbenzophenon, 2-Hydroxy-4-methoxybenzophenon, 2-Methyl-5-(2-oxo-3-bornylidenmethyl)sulfonsäure und deren Salze, 2-Phenylbenzimidazol-5-sulfonsäuresalze, 3-(4-(2,2-bis Ethoxycarbonylvinyl)-phenoxy)propenyl)-methoxysiloxan / Dimethylsiloxan - Copolymer, 3-(4-Methylbenzyliden)campher, 3-Benzylidencampher, 4-(2-Oxo-3-bornylidenmethyl)benzolsulfonsäure, 4-(2-Oxo-3-bornylidenmethyl)benzolsulfonsäuresalze, 4-(Dimethylamino)-benzoesäure(2-ethylhexyl)ester, 4-(Dimethylamino)benzoesäure-amylester, 4-(tert.-Butyl)-4'-methoxydibenzoylmethan, 4,4',4"-(1,3,5-Triazin-2,4,6-triyltriimino)-tris-benzoesäure-tris(2-ethylhexylester) (auch: 2,4,6-Tris-[anilino-(p-carbo-2'-ethyl-1'-hexyloxy)l-1,3,5-triazin (INCI: Ethylhexyl Triazone), 4-Dicyanomethylen-2,6-dimethyl-1,4-dihydropyridin-N-(ethyloxysulfatestersalz), 4-Dicyanomethylen-2,6-dimethyl-1,4-dihydropyridin-N-(ethyloxysulfatestersalz) das mono-Natriumsalz, 4-Isopropylbenzylsalicylat, 4-Methoxybenzalmal-säuredi(2-ethylhexyl)ester, 4-Methoxyzimtsäure(2-ethylhexyl)ester, 4-Methoxyzimtsäureisoamylester, Benzol-1,4-di(2-oxo-3-bornylidenmethyl-10-sulfonsäure), Benzophenone-3, Benzophenone-4, Bis-Ethylhexyloxyphenol Methoxyphenyl Triazine (Tinosorb® S), Bisoctrizole, Butyl Methoxydibenzoylmethane, Diethylamino Hydroxybenzoyl Hexyl Benzoate, Dimethicodiethylbenzalmalonat, Dioctylbutylamidotriazon (INCI: DiethylhexylButamidotriazone), Disodium Phenyldibenzimidazol Tetrasulfonate, Ethylhexyl Methoxycrylene, Ethylhexyl Salicylate (Octylsalicylat), Ethylhexylsalicylat, Homomenthylsalicylat, Homosalate, Isoamyl p-Methoxycinnamate, Polysilicone-15, Merocyanine, Methylene Bis-Benzotriazolyl Tetramethylbutylphenol, Titandioxid (mit und ohne Coating), Phenylbenzimidazole Sulfonic Acid, Phenylen-1,4-bis-(2-benzimidazyl)-3,3'-5,5'-tetrasulfonsäuresalze, Piperazinderivate, Polysilicone-15, Salicylsäure(2-ethylhexyl)ester, Salicylsäure(4-isopropylbenzyl)ester, Salicylsäurehomomenthylester, Terephthalidendicamphersulfonsäure, Terephthalidene Campher Sulfonsäure, Titandioxid und/oder Zinkoxid.

Die Pigmente (Titandioxid, Zinkoxid) können vorteilhaft im Sinne der vorliegenden Erfindung auch in Form kommerziell erhältlicher öliger oder wäßriger Vordispersionen zur Anwendung kommen. Diesen Vordispersionen können vorteilhaft Dispergierhilfsmittel und/oder Solubilisationsvermittler zugesetzt sein

Die Pigmente (Titandioxid, Zinkoxid) können erfindungsgemäß vorteilhaft oberflächlich behandelt ("gecoatet") sein, wobei beispielsweise ein hydrophiler, amphiphiler oder hydrophober Charakter gebildet werden bzw. erhalten bleiben soll. Diese Oberflächenbehandlung kann darin bestehen, dass die Pigmente nach an sich bekannten Verfahren mit einer dünnen hydrophilen und/oder hydrophoben anorganischen und/oder organischen Schicht versehen werden. Die verschiedenen Oberflächenbeschichtungen können im Sinne der vorliegenden Erfindung auch Wasser enthalten.

Anorganische Oberflächenbeschichtungen im Sinne der vorliegenden Erfindung können bestehen aus Aluminiumoxid ($Al_2O_3$), Aluminiumhydroxid $Al(OH)_3$, bzw. Aluminiumoxidhydrat (auch: Alumina, CAS-Nr.: 1333-84-2), Natriumhexametaphosphat ($(NaPO_3)_3$), Natriummetaphosphat ($(NaPO_3)_n$), Siliciumdioxid ($SiO_2$) (auch: Silica, CAS-Nr.: 7631-86-9), Bariumsulfat ($BaSO_4$) oder Eisenoxid ($Fe_2O_3$). Diese anorganischen Oberflächenbeschichtungen können allein, in Kombination und/oder in Kombination mit organischen Beschichtungsmaterialien vorkommen.

Organische Oberflächenbeschichtungen im Sinne der vorliegenden Erfindung können bestehen aus pflanzlichem oder tierischem Aluminiumstearat, pflanzlicher oder tierischer Stearinsäure, Laurinsäure, Dimethylpolysiloxan (auch: Dimethicone), Methylpolysiloxan (Methicone), Simethicone (einem Gemisch aus Dimethylpolysiloxan mit einer durchschnittlichen Kettenlänge von 200 bis 350 Dimethylsiloxan-Einheiten und Silicagel) oder Alginsäure. Diese organischen Oberflächenbeschichtungen können allein, in Kombination und/oder in Kombination mit anorganischen Beschichtungsmaterialien vorkommen.

Die Liste der genannten herkömmlichen UV-Filtersubstanzen, die im Sinne der vorliegenden Erfindung eingesetzt werden können, soll selbstverständlich nicht limitierend sein.

Besonders bevorzugte UV-Filter sind zu wählen aus der Gruppe Octocrylen, Homosalate, Ethylhexyl Salicylate (Octylsalicylat), Butyl Methoxydibenzoylmethane, Titanium Dioxide, Phenylbenzimidazole Sulfonic Acid, Bis-Ethylhexyloxyphenol Methoxyphenyl Triazine (Tinosorb® S), Polysilicone-15, Diethylamino Hydroxybenzoyl Hexyl Benzoate, Disodium Phenyldibenzimidazol Tetrasulfonate, Terephthalidene Campher Sulfonsäure, Ethylhexyl Triazone, Diethylhexylbutamidotriazone, 2-Ethylhexyl Methoxycinnamate, Isoamyl p-Methoxycinnamate,Polysilicone-15, Benzophenone-4, Methylene Bis-Benzotriazolyl Tetramethylbutylphenol,Titandioxid (mit und ohne Coating), Zinkoxid (mit und ohne Coating), 2,4,6-Tris-(biphenyl)-1,3,5-triazin und Benzophenone-3.

Ein hoher UV-Filteranteil bedeutet, dass der Anteil an UV-Lichtschutzsubstanzen über 5 Gew. %, bezogen auf die Gesamtmasse der Zubereitung, umfasst. Bevorzugt wird der Anteil im Bereich von 10 Gew. % bis 40 Gew. %, insbesondere zwischen 20 und 35 Gew. % gewählt. Bei einem SPF der Zubereitung von 30 umfasst die Zubereitung vorteilhaft einen UV-Filtersubstanzanteil von 20 bis 30 Gew. %, bezogen auf die Gesamtmasse der Zubereitung. Bei einem SPF der Zubereitung von 50 umfasst die Zubereitung vorteilhaft einen UV-Filtersubstanzanteil von 25 bis 35 Gew. %, bezogen auf die Gesamtmasse der Zubereitung.

Die Zubereitungen umfassen des Weiteren vorteilhaft ein oder mehrere Hautbefeuchtungsmittel mit einem Anteil bis 10 Gew.%, bevorzugt zwischen 2 und 7,5 Gew.%, besonders bevorzugt zwischen 2,5 und 5 Gew.%, bezogen auf die Gesamtmasse der Zubereitung.

Hautfeuchtigkeit ist ein Begriff aus der Kosmetikindustrie. Gesunde Haut besitzt eine natürliche Feuchtigkeit. Erst wenn die menschliche Haut Anomalien in Bezug auf Trockenheit aufweist, kommt mangelnde Hautfeuchtigkeit zum Tragen. Außer krankhaft bedingten Ursachen spielt auch das Alter der menschlichen Haut sowie die Pigmentierung eine Rolle. Eine wichtige Rolle bei der Feuchtigkeit der Haut spielen Feuchthaltefaktoren wie z.B. Urea. Diese können der Haut durch Hautpflegemittel zugeführt werden.

Normalerweise benötigt die menschliche Haut keinerlei Hilfsmittel zur Erhaltung der natürlichen Feuchtigkeit. Jedoch tragen ungesunde Lebensweise, trockene Luft (besonders in Solarien und geheizten bzw., klimatisierte Innenräumen), Umwelteinflüsse, Stress und lange Sonnenbäder zum Entzug von Feuchtigkeit bei. Auch lange und heiße Wannenbäder und Waschmittelreste in der Kleidung verursachen den Verlust der wichtigen Bestandteile des Hydrolipidsystems der Haut. Um einer Austrocknung der Haut vorzubeugen verwendet man Feuchtigkeitscremes. Auch rückfettende Seifen und weitgehend seifenfreie Waschmittel geben das Fett wieder an die Haut zurück. So wird sie wieder glatter und geschmeidiger. Hier werden insbesondere Körperreinigungsmittel empfohlen, die den natürlichen Säureschutzmantel der Haut nicht zerstören (pH der Haut = 5,5).

In manchen Wohnungen, deren Belüftung durch negative Umweltbedingungen nicht wie üblich möglich ist, können zur Unterstützung einer gesunden Hautfeuchtigkeit auch sogenannte Luftbefeuchter eingesetzt werden.

Als Befeuchtungsmittel, auch als Moisturiser bezeichnet, werden Stoffe oder Stoffgemische bezeichnet, welche kosmetischen oder dermatologischen Zubereitungen die Eigenschaft verleihen, nach dem Auftragen bzw. Verteilen auf der Hautoberfläche die Feuchtigkeitsabgabe der Hornschicht (auch transepidermal water loss (TEWL) genannt) zu reduzieren und/oder die Hydratation der Hornschicht positiv zu beeinflussen. Letzteres wird durch die Corneometrie gemessen, eine Methode die dem Experten hinreichend bekannt ist.

Vorteilhafte Feuchthaltemittel (Moisturizer) im Sinne der vorliegenden Erfindung sind beispielsweise Glycerin, Milchsäure und/oder Lactate, insbesondere Natriumlactat, Butylenglykol, Propylenglykol, Panthenol, Biosaccaride Gum-1, Glycine Soja, Harnstoff, Harnstoffderivate, Glycerylglucose,Ethylhexyloxyglycerin, Pyrrolidoncarbonsäure und Hyaluronsäure, Hyaloronsäurederivate, Aminosäuren und entsprechende Derivate, Glucosaminglycane, Honig. Ferner ist es insbesondere von Vorteil polymere Moisturizer aus der Gruppe der wasserlöslichen und/oder in Wasser quellbaren und/oder mit Hilfe von Wasser gelierbaren Polysaccharide zu verwenden. Insbesondere vorteilhaft sind beispielsweise ein fucosereiches Polysaccharid, welches in den Chemical Abstracts unter der Registraturnummer 178463-23-5 abgelegt und z.B. unter der Bezeichnung Fucogel®1000 von der Gesellschaft SOLABIA S.A. erhältlich ist. Moisturizer können vorteilhaft auch als Antifaltenwirkstoffe zum Schutz vor Hautveränderungen, wie sie z. B. bei der Hautalterung auftreten, verwendet werden.

Auch Salze sind sehr gute Befeuchtungsmittel. Letztere können beispielsweise gewählt werden aus der Gruppe der Salze mit Chlorid-Anionen, ferner anorganische Oxo-Element-Anionen. Auch auf organischen Anionen basierende Elektrolyte sind vorteilhaft, z.B. Lactate, Acetate, Benzoate, Citrate, Aminosäuren, Ethylendiamintetraessigsäure und deren Salze und andere mehr. Als Kationen der Salze werden bevorzugt Ammonium,- Alkalimetall-, Erdalkalimetall,- Magnesium-, Eisen- bzw. Zinkionen verwendet. Es bedarf an sich keiner Erwähnung, dass in Kosmetika nur physiologisch unbedenkliche Elektrolyte verwendet werden sollten. Besonders bevorzugt sind Kochsalz, Meersalz, Totes Meersalz, Magnesiumsulfat, Magnesiumchlorid, Zinksulfat und Mischungen daraus.

Bevorzugtes Befeuchtungsmittel ist Glycerin.

Weitere besonders bevorzugte Moisturiser sind zum Beispiel Polyole.

Als Polyole werden mehrwertige Alkohole, d.h. organische Verbindungen, die mindestens 2 alkoholische Hydroxylgruppen im Molekül tragen, bezeichnet. In einer Ausführungsform der Erfindung enthalten die Polyole 2 bis 6 Hydroxylgruppen pro Molekül. In einer Ausführungsform der Erfindung werden als Polyole niedermolekulare mehrwertige Alkohole eingesetzt, d.h. Verbindungen die 2 bis 18, insbesondere 2 bis 10, vorzugsweise 2 bis 6 C-Atome enthalten.

In einer bevorzugten Ausführungsform der Erfindung werden als Polyole Verbindungen eingesetzt, die mindestens 2 Hydroxylgruppen pro Molekül tragen und aus 2 bis 18, vorzugsweise 2 bis 10, insbesondere aus 2 bis 6 C-Atomen bestehen.

In einer bevorzugten Ausführungsform der Erfindung werden als Polyole Verbindungen eingesetzt, die 2 bis 6 Hydroxylgruppen pro Molekül tragen und aus 2 bis 6 C-Atomen bestehen.

Als Polyole können sowohl einzelne Polyole als auch Mischungen aus beliebigen Polyolen eingesetzt werden. Die Polyole können noch weitere funktionelle Gruppen, insbesondere Aminogruppen, enthalten bzw. mit Stickstoff modifiziert sein. In einer bevorzugten Ausführungsform enthalten die Polyole außer den Hydroxylgruppen keine weiteren funktionellen Gruppen.

Typische Beispiele für erfindungsgemäß einzusetzende Polyole sind Glycerin, Diglycerin, Triglycerin, Tetraglycerin, Alkylenglycole, wie beispielsweise Ethylenglycol, Diethylenglycol, Triethylenglykol, 1,2-Propylenglycol, 1,3-Propylenglycol, Butylenglycol, Hexylenglycol sowie Polyethylenglycole mit einem durchschnittlichen Molekulargewicht von 100 bis 1.000 Dalton; technische Oligoglyceringemische mit einem Eigenkondensationsgrad von 1,5 bis 10 wie etwa technische Diglyceringemische mit einem Diglyceringehalt von 40 bis 50 Gew.-%; Methylverbindungen, wie insbesondere Trimethylolethan, Trimethylolpropan, Trimethylolbutan, Pentaerythrit und Dipentaerythrit;

kurzkettige Alkyglucoside, insbesondere solche mit 1 bis 8 Kohlenstoffen im Alkylrest, wie beispielsweise Methyl- und Butylglucosid;

Zuckeralkohole mit 5 bis 12 Kohlenstoffatomen, wie beispielsweise Erythrit, Arabit, Adonit (Synonym Ribit), Xylit, Sorbit, Sorbitol, Mannit und Dulcit (Synonym Galactit).

Zucker mit 5 bis 12 Kohlenstoffatomen, wie beispielsweise Glucose oder Saccharose; Aminozucker, wie beispielsweise Glucamin; Dialkoholamine, wie Diethanolamin oder 2-Amino-1,3-propandiol.

Auch die 1,2 Alkandiole, insbesondere 1,2-Pentandiol, 1,2-Hexandiol und 1,2-Octandiol, sind vorteilhafte Hautbefeuchter. Letztgenannte werden bevorzugt in Einsatzkonzentrationen im Bereich zwischen 0,1 und 2 Gew. %, bevorzugt 0,2 bis 1 Gew. %, besonders bevorzugt 0,3 bis 0,75 Gew. %, bezogen auf die Gesamtmasse der Zubereitung, eingesetzt.

Als Anwendungsform der erfindungsgemäßen Zubereitungen seien z.B. genannt: Lösungen, Suspensionen, Emulsionen, PIT-Emulsionen, Hydrodispersionen, dünnflüssige Emulsion als Tränkungsmittel (z. B. Wipes), Pasten, Salben, Gele, Cremes, Lotionen, Aerosole und Sprays.

Bevorzugt handelt es sich bei den erfindungsgemäßen Zubereitungen um O/W-, W/O-, W/O/W-, O/W/O-Emulsionen und Hydrodispersionen, wobei O auch für Silikonöle stehen kann. Bevorzugt ist die Zubereitung auf Basis einer einer O/W-Emulsion oder Hydrodispersion formuliert.

Die erfindungsgemäßen Zubereitungen umfassen vorteilhaft keine Parabene, Formalgehydabspalter, organohalogenische Stoffe, Benzoesäure und deren Salze, Formiate und Teebaumöl sowie vorteilhaft auch kein Benzylalkohol und/oder Phenoxyethanol.

Diese Substanzen sind allesamt gängige Konservierungsmittel, weisen jedoch auch Nachteile auf. So ist DMDM Hydantoin ein Formaldehydabspalter. Formaldehyd ist wie allgemein bekannt eine giftige Substanz und ihr Gebrauch in Kosmetik nicht unumstritten. Benzylalkohol hat einen charakteristischen Geruch, welche in einer Formulierung mit Parfum maskiert werden muss. Parfuminhaltstoffe sind leider oft auch im Verdacht reizend oder sensibilisierend zu wirken. Deswegen ist es wünschenswert die Parfumkonzentration möglichst gering zu halten. Phenoxyethanol steht immer wieder im Verdacht Typ IV Kontaktallergien auszulösen und wird somit von Konsumenten zunehmend abgelehnt. Organische Halogenide sind vergleichsweise hochreaktive Stoffe und interagieren mit der Zellmembran und werden somit bei den Konsumenten ebenfalls stark abgelehnt. Teebaumöl ist nicht als Arzneimittel zugelassen und wird als Risikosubstanz für das Auftreten von Kontakt-Dermatitiden gewertet.

Die kosmetischen oder dermatologischen Zubereitungen gemäß der Erfindung können ferner kosmetische Hilfsstoffe und weitere Wirkstoffe enthalten, wie sie üblicherweise in solchen Zubereitungen verwendet werden, z. B. Konservierungsmittel, Konservierungshelfer, Bakterizide, Substanzen zum Verhindern des Schäumens, Farbstoffe und Farbpigmente, Verdickungsmittel, anfeuchtende und/oder feuchthaltende Substanzen, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösungsmittel oder Silikonderivate, Selbstbräuner, Puffer, pH Regulatoren, Pflanzliche Extrakte, Tenside, Treibgase, Puder, Sebumabsorbierende Substanzen, UV-Filter, Wirkstoffe wie zum Beispiel Anti Age, Anti-Cellulite, Anti Akne, Anti-Rosacea, Anti-Neurodermitis, Antioxidantien, Moisturiser, Chelatbildner, Antitraspirantien, Bleich und Färbemittel etc, sofern der Zusatz die geforderten Eigenschaften hinsichtlich Emulgatorgehalt, geforderter Stabilität und vor allem der Wasserfestigkeit nicht behindert.

Bevorzugte Lipidkomponenten in den erfindungsgemäßen Zubereitungen sind C 12-15 Alkylbenzoat, Isopropylpalmitat, Caprylic/Capric Triglyceride und/oder Octyldodecanol.

Bei der Herstellung der erfindungsgemäßen Zubereitungen zeigt sich ein weiterer unerwarteter Vorteil bei der Verwendung von Polyglyceryl-10-Stearat.

Bei der Emulsionsherstellung muss normalerweise eine Wasser- und eine Fettphase aufgeheizt werden, gemischt und diese Mischung anschließend wieder gekühlt werden. Das bedeutet eine mindestens zweimalige Zugabe von Wärmeenergie. Energetisch ist das ungünstig und ebenso problematisch bei ggf. thermisch labilen Wirkstoffen.

Überraschenderweise ist diese zweimalige Erwärmung durch die Verwendung von Polyglyceryl-10-Stearat als Emulgator nicht notwendig.

Emulsionszubereitungen mit PG-10-Stearat lassen sich im sogenannten cold-cold Verfahren herstellen. Normalerweise schmilzt man die Fettphase auf 80-100°C und heizt parallel die Wasserphase auf 80-100°C auf. Diese beiden heißen Phasen gibt man zusammen und rührt. Anschließend wird homogenisiert. In der Produktion muss dafür häufig gekühlt werden. Dieses Verfahren nennt man hot/hot-Verfahren. Cold/cold-Verfahren heißt also analog, man hat eine Wasserphase bei Raumtemperatur und eine Fettphase bei Raumtemperatur, welche man zusammen gibt und homogenisiert, was energetisch ein großer Vorteil ist.

Die erfindungsgemäßen Zubereitungen sind daher für die Einarbeitung thermisch labiler Wirkstoffe, wie beispielsweise natürlichen Ölen, Wirkstoffe, Vitaminen, Parfum oder auch einzelnen Parfuminhaltsstoffen bevorzugt.

Als thermisch labil bezeichnet man Substanzen, die beim Erwärmen über 50°C sich farblich, geruchlich oder in physikalischen Parametern verändern oder gar sich ganz oder teilweise abbauen.

Nachfolgende Beispiele illustrieren die erfindungsgemäßen Zubereitungen. Die angegebenen Anteile sind Gewichtsanteile, jeweils bezogen auf die Gesamtmasse der Zubereitung.

Beispiele

| O/W Emulsion | 1 | 2 | 3 | 4 | 5 |
|---|---|---|---|---|---|
| Polyglyceryl-10-stearat | 2 | 2 | 1 | 0,5 | 0,2 |
| Glyceryl Stearat SE | | 0,5 | 1 | 1 | 1 |
| Cetearylalkohol | | | 1,5 | 1 | |
| Stearylalkohol | 2 | 1,5 | | | |
| Acrylates/$C_{10-30}$ Alkyl Acrylat Crosspolymer | | 0,2 | | | 0,1 |
| Xanthan Gum | 0,4 | | 0,2 | 0,2 | 0,3 |
| $C_{12-15}$ Alkyl Benzoat | | 3 | | | 5 |
| Dicaprylyl Carbonat | | 2 | | | |

(fortgesetzt)

| O/W Emulsion | 1 | 2 | 3 | 4 | 5 |
|---|---|---|---|---|---|
| Myristylmyristat | | | 2 | | 1 |
| Butylenglycol Dicaprylat/Dicaprat | | | 3 | | 3 |
| Propylheptyl Caprylat | 5 | | 5 | 2 | |
| Dicaprylyl Ether | | | | | 2 |
| Octyldodecanol | 1 | | | | |
| Cyclopentasilxonan | | 5 | 5 | 1 | 10 |
| MT Propylsilsesquioxan Wachsharz mit M= Si (C 30+) (CH$_3$)$_2$ | 2 | 3 | 1 | 5 | 1,5 |
| Dimethicone | 6 | | | 5 | |
| Dimethiconol | | 5 | | | |
| 1-Methyl-1,3-propandiol | 5 | 8 | | | |
| Glycerin | 3 | 5 | 3 | 5 | 3 |
| Octan-1,2-diol | 2 | | 1 | | |
| Bis-Vinyl Dimethicon / PPG-20 Crosspolymer | | 4 | 1 | | |
| 2-(4'-Diethylamino-2'-hydoxybenzoyl)-benzoesäurehexylester | 3 | 5 | 1 | 0,5 | 2 |
| Octocrylen | | | 5 | | |
| Titandioxid | | 0,5 | 1 | | 2 |
| Phenylbenzimidazol Sulfonsäure | | | | 4 | 2 |
| Octylsalicylat | | | 5 | | |
| Polysilicon-15 | | | | | 2 |
| Ethylhexylmethoxycinnamat | | 10 | | | |
| Methylen Bis-Benzotriazolyl Tetramethylbutylphenol | 3 | | | 2 | |
| Ethylhexyltriazon | 3 | 2 | | | 3 |
| Tris-Triphenyl Triazine | | | 2 | | |
| Bis-Ethylhexylphenol Methoxyphenyl Triazin | | 2 | | | |
| PVP/Hexadecen Copolymer | | 0,5 | 0,1 | | |
| Vitamin E Acetat | 0,2 | 0,2 | 0,2 | 0,3 | 0,1 |
| Na$_2$H$_2$EDTA | 0,1 | 0,1 | | | 0,2 |
| Parfüm | 0,2 | 0,3 | 0,3 | 0,4 | 0,25 |
| Methylisothoazolinone | 0,05 | 0,1 | 0,05 | 0,1 | 0,6 |
| Ethylhexylglycerin | 0,25 | 0,25 | 0,5 | 0,5 | |
| Benzylalkohol | | | 0,5 | | 0,5 |
| Pentandiol | 1 | | | 0,5 | 1,5 |
| Methylpropandiol | | 3 | | | 3,5 |
| Natriumhydroxid | q.s. | q.s. | q.s. | q.s. | q.s. |
| Wasser | ad 100,0 | ad 100,0 | ad 100,0 | ad 100,0 | ad 100,0 |

(fortgesetzt)

| | 6 | 7 | 8 | 9 | 10 |
|---|---|---|---|---|---|
| Glyceryl Stearat SE | 2 | 0,5 | 3 | 0,5 | 0,2 |
| PG-10 Stearat | 1,5 | 0,5 | 0,1 | 1,0 | 1,5 |
| Polyglyceryl-3 Methylglycose Distearat | | | | 0,5 | 2,5 |
| Copolymer aus Vinylpyrrolidon und Acrylsäure | | 1,5 | | | |
| Stearylalkohol | | | | 2 | |
| Cetylalkohol | 2 | | | | 2 |
| Acrylates/$C_{10-30}$ Alkyl Acrylat Crosspolymer | | | 0,3 | 0,2 | 0,1 |
| Carbomer | | | 0,2 | | 0,1 |
| Xanthan Gum | 0,3 | | | | |
| $C_{12-15}$ Alkyl Benzoat | 3 | 5 | | | |
| Butylenglycol Dicaprylat/Dicaprat | | | 5 | 7 | |
| Dicaprylyl Carbonat | | 2 | | 2 | |
| Octyldodecanol | | | | | 2 |
| Cyclisches Silikon | | 2 | 10 | 5 | |
| MT Propylsilsesquioxan Wachsharz mit M= Si (C 30+) $(CH_3)_2$ | 2 | | | 5 | 3 |
| Lineares Silikon | 5 | 8 | 5 | | |
| Aluminium Starch Octenylsuccinat | | 0,5 | 1 | | |
| Glycerin | 2 | 4 | 5 | 10 | 8 |
| Ethanol | 3 | 4 | | | 4 |
| 2-(4'-Diethylamino-2'-hydoxybenzoyl)-benzoesäurehexylester | | 1,5 | | 0,5 | 6 |
| Octocrylen | 2,5 | | 6 | 5 | 7,5 |
| Butyl Methoxydibenzoylmethan | 2,5 | | 3 | 2 | |
| Ethylhexylmethoxycinnamat | | | | | 7,5 |
| Octylsalicylat | | 5 | | | |
| Phenylbenzimidazol Sulfonsäure | 1 | 1 | | | |
| Titandioxid | | | 5 | | 3 |
| Bis-Ethylhexylphenol Methoxyphenyl Triazin | | 2 | | | 2 |
| Isoamylmethoxycinnamat | 5 | | | | |
| Ethylhexyltriazin | | | | 2 | 2 |
| Terephthalydine Dicamphor Sulfonsäure | | 4 | | | |
| Vitamin E Acetat | 0,2 | 0,1 | 0,5 | 0,25 | 0,3 |
| $Na_2H_2EDTA$ | 0,2 | 0,2 | 0,2 | 0,2 | 0,5 |
| Stärke | 1 | | | 3 | |
| Parfüm | | 0,2 | 0,1 | 0,3 | 0,25 |
| Methylparaben | 0,4 | 0,3. | | 0,2 | 0,4 |
| Ethylparaben | 0,4 | 0,3 | | | |

(fortgesetzt)

| | 6 | 7 | 8 | 9 | 10 |
|---|---|---|---|---|---|
| Phenoxyethanol | 0,5 | 0,7 | 0,5 | | |
| Methylisothoazolinone | | | 0,5 | 0,5 | |
| Methylpropandiol | | | | | 3 |
| Caprylyl Glcol | | | | | 0,25 |
| Natriumhydroxid, Farbstoffe | q.s. | q.s. | q.s. | q.s. | q.s. |
| Wasser | Ad 100 | Ad 100 | Ad 100 | Ad 100 | Ad 100 |
| | **11** | **12** | **13** | **14** | **15** |
| Glycerylstearat | 0,2 | 2 | 0,75 | 1 | 0,5 |
| PG-10 Stearat | 2 | 0,25 | 1 | 1 | 1,25 |
| Cetearylalkohol | 4 | | | 2 | |
| Stearylalkohol | | 2 | 1 | | |
| Cetylalkohol | | | 1 | 1 | |
| Acrylates/$C_{10-30}$ Alkyl Acrylat Crosspolymer | | | 0,05 | 0,2 | 0,2 |
| Carbomer | 0,1 | | 0,2 | | |
| Xanthan Gum | | 0,3 | | | |
| Triheptanoin | | | 2 | | |
| $C_{12-15}$ Alkyl Benzoat | | 7 | | | 3 |
| Butylenglycol Dicaprylat/Dicaprat | 2 | 4 | | | 5 |
| Dicaprylyl Carbonat | 4 | | | | |
| Phenylethylbenzoat | | | 5 | 5 | 4 |
| Diisopropylsebacat | | 3 | | 5 | |
| Cyclisches Silikon | 3 | | | | |
| 2-Propylheptyloctanoat | 4 | | 3 | | 2 |
| MT Propylsilsesquioxan Wachsharz mit M= Si (C 30+) $(CH_3)_2$ | 2 | | 8 | | 3 |
| Glycerin | 7,5 | 5 | 10 | 3 | 5 |
| Ethanol | | | 2 | | 4 |
| Bis-Ethylhexyloxyphenol Methoxyphenyltriazin | 2 | 3 | 0,5 | 1,5 | 3 |
| Ethylhexylmethoxycinnamat | | | 7 | 1 | 4 |
| Octylsalicylat | | | | | 5 |
| Homosalat | | | 3 | | |
| Octocrylen | 5 | | | 4 | |
| Phenylbenzimidazol Sulfonsäure | 1 | | | 2 | |
| Butyl Methoxydibenzoylmethan | 2 | | 3 | 4 | 1 |
| 2,4,6-Tris-(biphenyl)-1,3,5-triazin | | 2 | | | 1 |
| Diethylhexylbutamidotriazin | | | 2 | 1,5 | |
| Ethylhexyltriazin | | | | 1,5 | |
| Drometrizole Trisiloxane | | 2 | | | |

(fortgesetzt)

|  | 11 | 12 | 13 | 14 | 15 |
|---|---|---|---|---|---|
| Tapiokastärke | 1 |  | 2,5 |  |  |
| Natrium-Stärke Octenylsuccinat |  |  |  | 1 |  |
| Na$_2$H$_2$EDTA | 0,1 |  |  |  |  |
| Parfüm | 0,2 | 0,3 | 0,4 | 0,5 |  |
| Parabene | 0,5 |  |  |  |  |
| Natriumhydroxid, Farbstoffe | q.s. | q.s. | q.s. | q.s. | q.s. |
| Ethylhexylglycerin |  | 1 | 1 | 0,5 | 1 |
| Caprylylglykol |  | 1 |  | 0,5 | 1 |
| Ethyllauroylarginate 20% in Glycerin | 1,8 | 1,8 | 1,8 | 1,8 | 0,05 |
| Phenoxyethanol | 0,5 | 0,5 | 0,5 | 0,5 |  |
| Wasser | ad 100,0 | ad 100,0 | ad 100,0 | ad 100,0 | ad 100,0 |
|  | **16** | **17** | **18** | **19** | **20** |
| Glycerylstearat | 0,2 | 2 | 1 | 2 | 1 |
| PG-10 Stearat | 2 | 0,25 | 1 | b,25 | 1 |
| Cetearylalkohol | 4 |  | 2 |  | 2 |
| Stearylalkohol |  | 2 |  | 2 |  |
| Cetylalkohol |  |  | 1 |  | 1 |
| Acrylates/C$_{10-30}$ Alkyl Acrylat Crosspolymer |  |  | 0,2 |  | 0,2 |
| Carbomer | 0,1 |  |  |  |  |
| Xanthan Gum |  | 0,3 |  | 0,3 |  |
| Triheptanoin |  |  |  |  |  |
| C$_{12-15}$ Alkyl Benzoat |  | 7 |  | 7 |  |
| Butylenglycol Dicaprylat/Dicaprat | 2 | 4 |  | 4 |  |
| Dicaprylyl Carbonat | 4 |  |  |  |  |
| Phenylethylbenzoat |  |  | 5 |  | 5 |
| Diisopropylsebacat |  | 3 | 5 | 3 | 5 |
| Cyclisches Silikon | 3 |  |  |  |  |
| 2-Propylheptyloctanoat | 4 |  |  |  |  |
| MT Propylsilsesquioxan Wachsharz mit M= Si (C 30+) (CH$_3$)$_2$ | 2 |  |  |  |  |
| Glycerin | 7,5 | 5 | 3 | 5 | 3 |
| Ethanol |  |  | 3 | 3 |  |
| Ethylhexyl Methoxycinnamate | 7,00 | 7,00 | 7,00 | 7,00 | 7,00 |
| Octocrylene | 2,80 | 2,80 | 2,80 | 2,80 | 2,80 |
| Butyl Methoxydibenzoylmethane | 3,00 | 3,00 | 3,00 | 3,00 | 3,00 |
| Benzotriazolyl Dodecyl p-Cresol | 9,00 |  |  |  |  |
| Diethylhexyl 2,6-Naphthalate |  | 9,00 |  |  | 4,5 |
| Zinkoxide |  |  | 5 |  | 25 |

(fortgesetzt)

| | 16 | 17 | 18 | 19 | 20 |
|---|---|---|---|---|---|
| Ethylmethylmethoxycrylen | | | 6 | 6 | |
| Titaniumdioxide | | | | 5 | 5 |
| Silica | | | 0,5 | | |
| Na$_2$H$_2$EDTA | 0,1 | | | | |
| Parfüm | 0,2 | 0,3 | 0,5 | 0,3 | 0,5 |
| Natriumhydroxid, Farbstoffe | q.s. | q.s. | q.s. | q.s. | q.s. |
| Ethylhexylglycerin | 1 | | | | |
| Caprylylglykol | | 1 | 0,5 | 1 | 0,5 |
| Piroctone Olamine | | | 0,05 | | 0,05 |
| Phenoxyethanol | 0,5 | | | | |
| Wasser | ad 100,0 | ad 100,0 | ad 100,0 | ad 100,0 | ad 100,0 |

**Patentansprüche**

1. Wasserfeste kosmetische oder dermatologische Lichtschutzzubereitung umfassend ein oder mehrere UV-Filtersubstanzen, 0,1 bis weniger als 6 Gew.% Polyglyceryl-10-Stearat, bezogen auf die Gesamtmasse der Zubereitung, **dadurch gekennzeichnet, dass** die Zubereitung keine Lecithine enthält und O/W-Emulsionszubereitungen 4 und 5 bestehend aus

| | 4 | 5 |
|---|---|---|
| Panthenol | 0,7 | 0 |
| Butylenglykol | 5 | 10 |
| Propylenglykol | 5 | --- |
| Benzethoniumchlorid | 0,5 | 0,9 |
| Lauroylethylarginat | 1,0 | 0,5 |
| medizinisches Weissöl | 0 | 0 |
| Isopropylpalmitat | 0 | 0 |
| Caprylic/Capric Triglycerid | 0 | 1,00 |
| Cetearylalkohol | 2,5 | 4,00 |
| Cetylalkohol | 0 | 0 |
| lineares Silikonöl | 0 | 0,50 |
| cyclisches Silikonöl | 0 | 0 |
| C12-15 Alkylbenzoat | 0 | 2,00 |
| Sheabutter | 0 | 0 |
| Dicaprylylether | 10 | 0 |
| Dicaprylylcarbonat | 0 | 0 |
| Glyceryl stearat | 2,4 | 2,4 |
| Tapiocastärke | 0 | 0 |
| Glycerin | 8 | 8 |
| Butylenglykol | 1 | 0 |

(fortgesetzt)

| | 4 | 5 |
|---|---|---|
| Zitronensäure | 0 | 0 |
| Natronlauge 45% | 0,25 | 1 |
| Polyglyceryl-10 Myristat | 0 | 0 |
| Polyglyceryl-10 Stearat | 1 | 1 |
| Polyacrylsäure, Na-Salz (Carbopol 981) | 0 | 0 |
| Acrylic Acid/VP Crosspolymer | 0,5 | 0,75 |
| Trinatrium EDTA | | 1 |
| Ethylhexylmethoxycinnamat | 2 | 0 |
| Butylmethoxydibenzoylmethan | 0 | 2 |
| Phenylbenzimidazolesulfonsäure, Natrium-Salz | 0 | 2 |
| Ethylhexylsalicylat | 0 | 2 |
| Titandioxid, silikongecoated | 0,3600 | 0 |
| Octocrylen | 0 | 2 |
| Parfum | 0 | 0,20 |
| Wasser | ad 100 | ad 100 |

sowie O/W-Emulsionszubereitungen 49 und 50 bestehend aus

| | 49 | 50 |
|---|---|---|
| Panthenol | 0,7 | 0 |
| Butylenglykol | 5 | 10 |
| Propylenglykol | 5 | --- |
| Methylisothiazolinone | 0,06 | --- |
| Benzethoniumchlorid | 0,15 | --- |
| Piroctonolamin | 0,15 | --- |
| Lauroylethylarginat | 0,15 | 1,75 |
| medizinisches Weißöl | 0 | 0 |
| Isopropylpalmitat | 0 | 0 |
| Caprylic/Capric Triglycerid | 0 | 1,00 |
| Cetearylalkohol | 2,5 | 4,00 |
| Cetylalkohol | 0 | 0 |
| lineares Silikonöl | 0 | 0,50 |
| zyklisches Silikonöl | 0 | 0 |
| C12-15 Alkylbenzoat | 0 | 2,00 |
| Sheabutter | 0 | 0 |
| Dicaprylylether | 10 | 0 |
| Dicaprylylcarbonat | 0 | 0 |
| Glyceryl Stearate | 2,4 | 2,4 |

(fortgesetzt)

| | 49 | 50 |
|---|---|---|
| Tapiocastärke | 0 | 0 |
| Glycerin | 8 | 8 |
| Zitronensäure | 0 | 0 |
| Natronlauge 45%ig | 0,25 | 1 |
| Polyglyceryl-10 Myristat | 0 | 0 |
| Polyglyceryl-10 Stearat | 1 | 1 |
| Polyacrylsäure, Na-Salz | 0 | 0 |
| Acrylic Acid/VP Crosspolymer | 0,5 | 0,75 |
| Trinatrium EDTA | | 1 |
| Ethylhexylmethoxycinnamat | 2 | 0 |
| Butylmethoxydibenzoylmethan | 0 | 2 |
| Phenylbenzimidazolesulfonsäure | 0 | 2 |
| Ethylhexylsalicylat | 0 | 2 |
| Titandioxid+ Trimethoxycaprylylsilan | 0,3600 | 0 |
| Octocrylen | 0 | 2 |
| Parfum | 0 | 0,20 |
| Wasser | ad 100 | ad 100 |

ausgenommen sind, wobei die Zahlenwerte Gewichtsanteile bezogen auf die Gesamtmasse der Zubereitungen darstellen.

2. Zubereitung nach Anspruch 1 umfassend ein oder mehrere Filmbildner.

3. Zubereitung nach Anspruch 1 umfassend 0,1 bis 2,8 Gew. % Polyglyceryl-10-Stearat, bezogen auf die Gesamtmasse der Zubereitung.

4. Zubereitung nach einem der vorstehenden Ansprüchen umfassend neben Polyglyceryl-10-Stearat keine weiteren Emulgatoren.

5. Zubereitung nach Anspruch 1, 2 oder 3, umfassend neben Polyglyceryl-10-Stearat ein oder mehrere Emulgatoren, die bei 25°C fest, pastös oder flüssig und nicht ethoxiliert sind.

6. Zubereitung nach Anspruch 5 umfassend als zusätzlichen Emulgator Glycerylstearat.

7. Zubereitung nach einem der vorstehenden Ansprüche als O/W-Emulsion.

8. Zubereitung nach einem der Ansprüche 1 bis 6 als Hydrodispersion.

9. Zubereitung nach einem der vorstehenden Ansprüche umfassend ein oder mehrere UV-Filter gewählt aus der Gruppe Octocrylen, Homosalate, Ethylhexyl Salicylate (Octylsalicylat), Butyl Methoxydibenzoylmethane, Titanium Dioxide, Phenylbenzimidazole Sulfonic Acid, Bis-Ethylhexyloxyphenol Methoxyphenyl Triazine, Polysilicone-15, Diethylamino Hydroxybenzoyl Hexyl Benzoate, Disodium Phenyldibenzimidazol Tetrasulfonate, Terephthalidene Campher Sulfonsäure, Ethylhexyl Triazone, Diethylhexylbutamidotriazone, 2-Ethylhexyl Methoxycinnamate, Isoamyl p-Methoxycinnamate, Benzophenone-4, Methylene Bis-Benzotriazolyl Tetramethylbutylphenol,Titandioxid (mit und ohne Coating), Zinkoxid (mit und ohne Coating), 2,4,6-Tris-(biphenyl)-1,3,5-triazin und Benzophenone-3.

10. Zubereitung nach einem der vorstehenden Ansprüche umfassend ein oder mehrere Hautbefeuchtungsmittel.

20

**11.** Zubereitung nach einem der vorstehenden Ansprüche umfassend keine Parabene, Formalgehydabspalter, organohalogenische Stoffe, Benzoesäure und deren Salze, Formiate und Teebaumöl.

**Claims**

**1.** Water-resistant cosmetic or dermatological photoprotective preparation comprising one or more UV filter substances, 0.1 to less than 6% by weight of polyglyceryl-10 stearate, based on the total mass of the preparation, **characterized in that** the preparation comprises no lecithins and O/W emulsion preparations 4 and 5 consisting of

|  | 4 | 5 |
|---|---|---|
| Panthenol | 0.7 | 0 |
| Butylene glycol | 5 | 10 |
| Propylene glycol | 5 | -- |
| Benzethonium chloride | 0.5 | 0.9 |
| Lauroyl ethyl arginate | 1.0 | 0.5 |
| Medicinal white oil | 0 | 0 |
| Isopropyl palmitate | 0 | 0 |
| Caprylic/capric triglyceride | 0 | 1.00 |
| Cetearyl alcohol | 2.5 | 4.00 |
| Cetyl alcohol | 0 | 0 |
| Linear silicone oil | 0 | 0.50 |
| Cyclic silicone oil | 0 | 0 |
| C12-15 alkyl benzoate | 0 | 2.00 |
| Shea butter | 0 | 0 |
| Dicaprylyl ether | 10 | 0 |
| Dicaprylyl carbonate | 0 | 0 |
| Glyceryl stearate | 2.4 | 2.4 |
| Tapioca starch | 0 | 0 |
| Glycerol | 8 | 8 |
| Butylene glycol | 1 | 0 |
| Citric acid | 0 | 0 |
| Sodium hydroxide solution 45% | 0.25 | 1 |
| Polyglyceryl-10 myristate | 0 | 0 |
| Polyglyceryl-10 stearate | 1 | 1 |
| Polyacrylic acid, Na salt (Carbopol 981) | 0 | 0 |
| Acrylic acid/VP crosspolymer | 0.5 | 0.75 |
| Trisodium EDTA |  | 1 |
| Ethylhexyl methoxycinnamate | 2 | 0 |
| Butylmethoxydibenzoylmethane | 0 | 2 |
| Phenylbenzimidazolesulfonic acid, sodium salt | 0 | 2 |
| Ethylhexyl salicylate | 0 | 2 |
| Titanium dioxide, silicone-coated | 0.3600 | 0 |

# EP 2 775 997 B1

(continued)

|  | 4 | 5 |
|---|---|---|
| Octocrylene | 0 | 2 |
| Perfume | 0 | 0.20 |
| Water | ad 100 | ad 100 |

and O/W emulsion preparations 49 and 50 consisting of

|  | 49 | 50 |
|---|---|---|
| Panthenol | 0.7 | 0 |
| Butylene glycol | 5 | 10 |
| Propylene glycol | 5 | |
| Methylisothiazolinones | 0.06 | - |
| Benzethonium chloride | 0.15 | - |
| Piroctone olamine | 0.15 | - |
| Lauroyl ethyl arginate | 0.15 | 1.75 |
| Medicinal white oil | 0 | 0 |
| Isopropyl palmitate | 0 | 0 |
| Caprylic/capric triglyceride | 0 | 1.00 |
| Cetearyl alcohol | 2.5 | 4.00 |
| Cetyl alcohol | 0 | 0 |
| Linear silicone oil | 0 | 0.50 |
| Cyclic silicone oil | 0 | 0 |
| C12-15 alkyl benzoate | 0 | 2.00 |
| Shea butter | 0 | 0 |
| Dicaprylyl ether | 10 | 0 |
| Dicaprylyl carbonate | 0 | 0 |
| Glyceryl stearate | 2.4 | 2.4 |
| Tapioca starch | 0 | 0 |
| Glycerol | 8 | 8 |
| Citric acid | 0 | 0 |
| Sodium hydroxide solution 45% strength | 0.25 | 1 |
| Polyglyceryl-10 myristate | 0 | 0 |
| Polyglyceryl-10 stearate | 1 | 1 |
| Polyacrylic acid, Na salt | 0 | 0 |
| Acrylic acid/VP crosspolymer | 0.5 | 0.75 |
| Trisodium EDTA | | 1 |
| Ethylhexyl methoxycinnamate | 2 | 0 |
| Butylmethoxydibenzoylmethane | 0 | 2 |
| Phenylbenzimidazolesulfonic acid | 0 | 2 |

22

(continued)

|  | 49 | 50 |
|---|---|---|
| Ethylhexyl salicylate | 0 | 2 |
| Titanium dioxide + trimethoxycaprylylsilane | 0.3600 | 0 |
| Octocrylene | 0 | 2 |
| Perfume | 0 | 0.20 |
| Water | ad 100 | ad 100 |

are excluded, where the numerical values are fractions by weight, based on the total mass of the preparation.

2. Preparation according to Claim 1, comprising one or more film formers.

3. Preparation according to Claim 1, comprising 0.1 to 2.8% by weight of polyglyceryl-10 stearate, based on the total mass of the preparation.

4. Preparation according to one of the preceding claims, comprising no further emulsifiers besides polyglycerol-10 stearate.

5. Preparation according to Claim 1, 2 or 3, comprising, besides polyglyceryl-10 stearate, one or more emulsifiers which, at 25°C, are solid, pasty or liquid and not ethoxylated.

6. Preparation according to Claim 5, comprising glyceryl stearate as additional emulsifier.

7. Preparation according to one of the preceding claims as O/W emulsion.

8. Preparation according to one of Claims 1 to 6 as hydrodispersion.

9. Preparation according to one of the preceding claims, comprising one or more UV filters selected from the group octocrylene, homosalate, ethylhexyl salicylate (octyl salicylate), butylmethoxydibenzoylmethane, titanium dioxide, phenylbenzimidazolesulfonic acid, bisethyl-hexyloxyphenol methoxyphenyltriazine, polysilicone-15, diethylaminohydroxybenzoyl hexylbenzoate, disodiumphenyldibenzimidazoleatetrasulfonate, terephthalidenecamphorsulfonic acid, ethylhexyltriazone, diethylhexylbutamidotriazone, 2-ethylhexylmethoxycinnamate, isoamyl p-methoxycinnamate, benzophenone-4, methylene-bisbenzotriazolyltetramethylbutylphenol, titanium dioxide (with and without coating), zinc oxide (with and without coating), 2,4,6-tris(biphenyl)-1,3,5-triazine and benzophenone-3.

10. Preparation according to one of the preceding claims, comprising one or more skin moisturizers.

11. Preparation according to one of the preceding claims, comprising no parabens, formaldehyde donors, organohalic substances, benzoic acid and salts thereof, formates and tea tree oil.

## Revendications

1. Préparation photoprotectrice cosmétique ou dermatologique résistante à l'eau, comprenant une ou plusieurs substances de filtration des UV, 0,1 à moins de 6 % en poids de stéarate de polyglycéryle-10, par rapport à la masse totale de la préparation, **caractérisée en ce que** la préparation ne contient pas de lécithine, et les préparations d'émulsion H/E 4 et 5 constituées par :

|  | 4 | 5 |
|---|---|---|
| Panthénol | 0,7 | 0 |
| Butylène glycol | 5 | 10 |
| Propylène glycol | 5 | - |

(suite)

|  | 4 | 5 |
|---|---|---|
| Chlorure de benzéthonium | 0,5 | 0,9 |
| Arginate de lauroyléthyle | 1,0 | 0,5 |
| Huile blanche médicale | 0 | 0 |
| Palmitate d'isopropyle | 0 | 0 |
| Triglycéride caprylique/caprique | 0 | 1,00 |
| Alcool cétéarylique | 2,5 | 4,00 |
| Alcool cétylique | 0 | 0 |
| Huile de silicone linéaire | 0 | 0,50 |
| Huile de silicone cyclique | 0 | 0 |
| Benzoate d'alkyle en C12-15 | 0 | 2,00 |
| Beurre de karité | 0 | 0 |
| Ether dicaprylylique | 10 | 0 |
| Carbonate dicaprylylique | 0 | 0 |
| Stéarate de glycéryle | 2,4 | 2,4 |
| Amidon de tapioca | 0 | 0 |
| Glycérine | 8 | 8 |
| Butylène glycol | 1 | 0 |
| Acide citrique | 0 | 0 |
| Soude caustique 45 % | 0,25 | 1 |
| Myristate de polyglycéryle-10 | 0 | 0 |
| Stéarate de polyglycéryle-10 | 1 | 1 |
| Acide polyacrylique, sel de Na (Carbopol 981) | 0 | 0 |
| Acrylic Acid/VP Crosspolymer | 0,5 | 0,75 |
| EDTA trisodium |  | 1 |
| Méthoxycinnamate d'éthylhexyle | 2 | 0 |
| Butylméthoxydibenzoylméthane | 0 | 2 |
| Acide phénylbenzimidazole-sulfonique, sel de sodium | 0 | 2 |
| Salicylate d'éthylhexyle | 0 | 2 |
| Dioxyde de titane, revêtement de silicone | 0,3600 | 0 |
| Octocrylène | 0 | 2 |
| Parfum | 0 | 0,20 |
| Eau | Jusqu'à 100 | Jusqu'à 100 |

ainsi que les préparations d'émulsion H/E 49 et 50 constituées par :

|  | 49 | 50 |
|---|---|---|
| Panthénol | 0,7 | 0 |
| Butylène glycol | 5 | 10 |

(suite)

| | 49 | 50 |
|---|---|---|
| Propylène glycol | 5 | --- |
| Méthylisothiazolinone | 0,06 | --- |
| Chlorure de benzéthonium | 0,15 | --- |
| Piroctonolamine | 0,15 | --- |
| Arginate de lauroyléthyle | 0,15 | 1,75 |
| Huile blanche médicale | 0 | 0 |
| Palmitate d'isopropyle | 0 | 0 |
| Triglycéride caprylique/caprique | 0 | 1,00 |
| Alcool cétéarylique | 2,5 | 4,00 |
| Alcool cétylique | 0 | 0 |
| Huile de silicone linéaire | 0 | 0,50 |
| Huile de silicone cyclique | 0 | 0 |
| Benzoate d'alkyle en C12-15 | 0 | 2,00 |
| Beurre de karité | 0 | 0 |
| Ether dicaprylylique | 10 | 0 |
| Carbonate dicaprylylique | 0 | 0 |
| Stéarate de glycéryle | 2,4 | 2,4 |
| Amidon de tapioca | 0 | 0 |
| Glycérine | 8 | 8 |
| Acide citrique | 0 | 0 |
| Soude caustique 45 % | 0,25 | 1 |
| Myristate de polyglycéryle-10 | 0 | 0 |
| Stéarate de polyglycéryle-10 | 1 | 1 |
| Acide polyacrylique, sel de Na | 0 | 0 |
| Acrylic Acid/VP Crosspolymer | 0,5 | 0,75 |
| EDTA trisodium | | 1 |
| Méthoxycinnamate d'éthylhexyle | 2 | 0 |
| Butylméthoxydibenzoylméthane | 0 | 2 |
| Acide phénylbenzimidazole-sulfonique | 0 | 2 |
| Salicylate d'éthylhexyle | 0 | 2 |
| Dioxyde de titane + triméthoxycaprylylsilane | 0,3600 | 0 |
| Octocrylène | 0 | 2 |
| Parfum | 0 | 0,20 |
| Eau | Jusqu'à 100 | Jusqu'à 100 |

étant exclues, les valeurs numériques désignant les proportions en poids relatives à la masse totale des préparations.

2. Préparation selon la revendication 1, comprenant un ou plusieurs agents filmogènes.

**3.** Préparation selon la revendication 1, comprenant 0,1 à 2,8 % en poids de stéarate de polyglycéryle-10, par rapport à la masse totale de la préparation.

**4.** Préparation selon l'une quelconque des revendications précédentes, ne comprenant pas d'autres émulsifiants que le stéarate de polyglycéryle-10.

**5.** Préparation selon la revendication 1, 2 ou 3, comprenant, en plus du stéarate de polyglycéryle-10, un ou plusieurs émulsifiants qui sont solides, pâteux ou liquides et non éthoxylés à 25 °C.

**6.** Préparation selon la revendication 5, comprenant du stéarate de glycéryle en tant qu'émulsifiant supplémentaire.

**7.** Préparation selon l'une quelconque des revendications précédentes, sous la forme d'une émulsion H/E.

**8.** Préparation selon l'une quelconque des revendications 1 à 6, sous la forme d'une hydrodispersion.

**9.** Préparation selon l'une quelconque des revendications précédentes, comprenant un ou plusieurs filtres UV choisis dans le groupe constitué par l'octocrylène, l'homosalate, le salicylate d'éthylhexyle (salicylate d'octyle), le butylméthoxydibenzoylméthane, le dioxyde de titane, l'acide phénylbenzimidazole-sulfonique, la bis-éthylhexyloxyphénol-méthoxyphényl-triazine, la polysilicone-15, le benzoate de diéthylamino-hydroxybenzoyl-hexyle, le phényldibenzimidazole-tétrasulfonate de disodium, l'acide téréphtalidène-camphre-sulfonique, l'éthylhexyl-triazone, la diéthylhexylbutamidotriazone, le méthoxycinnamate de 2-éthylhexyle, le p-méthoxycinnamate d'isoamyle, la benzophénone-4, le méthylène bis-benzotriazolyl-tétraméthylbutylphénol, le dioxyde de titane (avec .et sans revêtement), l'oxyde de zinc (avec et sans revêtement), la 2,4,6-tris-(biphényl)-1,3,5-triazine et la benzophénone-3.

**10.** Préparation selon l'une quelconque des revendications précédentes, comprenant un ou plusieurs agents d'hydratation de la peau.

**11.** Préparation selon l'une quelconque des revendications précédentes, ne comprenant pas de parabènes, de cliveurs de formaldéhyde, de substances organohalogénées, d'acide benzoïque et ses sels, de formiates et d'huile d'arbre à thé.

Abbildung 1

Konsistenz

**IN DER BESCHREIBUNG AUFGEFÜHRTE DOKUMENTE**

**In der Beschreibung aufgeführte Patentdokumente**

- WO 2003082182 A
- JP 2005162664 B
- US 5925615 A
- DE 19547679 A1
- DE 19724587 A1
- WO 0027197 A1
- DE 102008028821 A1
- DE 102008028822 A1
- DE 10213955 A1
- US 20060018853 A
- DE 2909087

**In der Beschreibung aufgeführte Nicht-Patentliteratur**

- *CHEMICAL ABSTRACTS,* 79777-30-3
- Contact angle measurement-a reliable supportive method for screening water-restistance of ultraviolet-protecting products in vivo. *Intern. Journal of Cosmetic Science,* 2007, vol. 29, 283-291
- *CHEMICAL ABSTRACTS,* 288254-16-0
- *CHEMICAL ABSTRACTS,* 1333-84-2
- *CHEMICAL ABSTRACTS,* 7631-86-9
- *CHEMICAL ABSTRACTS,* 178463-23-5